# EUROPEAN PATENT SPECIFICATION

(11) **EP 2 124 603 B1**
(45) Date of publication and mention of the grant of the patent: **19.11.2014**
(21) Application number: 07862429.3
(22) Date of filing: 03.12.2007
(51) Int. Cl.: A23C 9/12, A23C 21/02, A23J 3/34, A61K 8/64, A23L 1/305, A61Q 19/02

(54) **MILK PROTEIN HYDROLYZATES WITH REDUCED IMMUNOGENIC POTENTIAL**
MILCHPROTEINHYDROLYSATE MIT VERRINGERTEM IMMUNOGENEM POTENTIAL
HYDROLYSATS DE PROTÉINES DE LAIT PRÉSENTANT UN POTENTIEL IMMUNOGÉNIQUE RÉDUIT

(30) Priority: 20.12.2006 US 875967 P
(43) Date of publication of application: 02.12.2009
(73) Proprietor: DuPont Nutrition Biosciences ApS, 1001 Copenhagen K (DK)
(72) Inventor: KUMAR, Manoj, Palo Alto, California 94304 (US); WONG, David, Palo Alto, California 94304 (US)
(74) Representative: Williams, Aylsa
(86) International application number: PCT/US2007/024723
(87) International publication number: WO 2008/088472

(56) References cited:
- US-A- 5 866 357
- NAKAMURA T ET AL: "ENZYMATIC PRODUCTION OF HYPOALLERGENIC PEPTIDES FROM CASEIN" MILCHWISSENSCHAFT, VV GMBH VOLKSWIRTSCHAFTLICHER VERLAG. MUNCHEN, DE, vol. 48, no. 1, 1 January 1993 (1993-01-01), pages 11-14, XP000356112 ISSN: 0026-3788
- HOST A ET AL: "Hypoallergenic formulas - when, to whom and how long: after more than 15 years we know the right indication!" ALLERGY (OXFORD), vol. 59, no. Suppl. 78, August 2004 (2004-08), pages 45-52, XP009100976 ISSN: 0105-4538
- LINDA MONACI ET AL: "Milk allergens, their characteristics and their detection in food: A review" EUROPEAN FOOD RESEARCH AND TECHNOLOGY ; ZEITSCHRIFT FÜR LEBENSMITTELUNTERSUCHUNG UND -FORSCHUNG A, SPRINGER-VERLAG, BE, vol. 223, no. 2, 15 February 2006 (2006-02-15), pages 149-179, XP019420433 ISSN: 1438-2385
- GUADIX ET AL: "Production of whey protein hydrolysates with reduced allergenicity in a stable membrane reactor" JOURNAL OF FOOD ENGINEERING, BARKING, ESSEX, GB, vol. 72, no. 4, 1 February 2006 (2006-02-01), pages 398-405, XP005080364 ISSN: 0260-8774

## Description

### FIELD OF THE INVENTION

This relates to compositions containing hydrolyzed milk proteins and/or whey proteins, and methods for making them. Also disclosed are proteolytic hydrolyzates of such proteins having reduced immunogenic potential for use in applications where there is risk of undesirable immunological response to the proteins.

### BACKGROUND

Milk is a product used widely not only as a nutritious beverage in its fluid form, but also as a base for numerous other products such as ice-cream, yogurt, butter, cream, and cheese. In addition, milk and components thereof are used widely as nutritional and functional ingredients in many food products.

Whey is a by-product of cheese production. Whey has become a useful food ingredient or additive for various purposes. A typical cheese production process may produce only about 10 pounds of cheese, but 90 pounds of whey for each 100 pounds of milk processed. Whey is high in lactose, a naturally-occurring milk sugar, and also contains a number of proteins that do not coagulate or precipitate during the cheese-making process, but rather remain soluble. Whey proteins are known to be highly nutritious and can thus be valuable, but are present in a dilute aqueous solution of salts, lactose, proteins, and some lipids.

Because of its dilute nature, in some locations whey is still frequently treated as a waste product or effluent, despite the expense of disposing of it. Where economically feasible, whey is frequently spray-dried, or further processed to separate the proteins and/or the lactose from the water, and from each other.

Whey, or various components thereof, can be added to a large variety of processed foods to provide excellent nutritional and functional properties for both the processor and consumer.

Whey proteins may comprise about 20% of the total milk protein. Whey composition derived after "curding" in cheese production includes proteins, along with the lactose, fat, and ash (2-5%). Lactalbumin and lactoglobulin are two milk proteins prominent in whey. Each of these proteins, as well as other proteins found in milk, have been reported to have immunogenic potential, for example as allergens. Because these proteins are potential immunogens or even allergens, certain consumers may avoid the use of food and/or cosmetic products containing milk, whey, or components thereof, for example the whey proteins lactalbumin and lactoglobulin. In some circumstances medical professional may advise certain consumers to avoid products containing milk or whey proteins. Thus, there is an ongoing need for reducing the immunogenic potential of milk proteins and whey proteins.

### SUMMARY

In several of its various aspects, compositions and methods are provided for using compositions of containing hydrolyzed milk proteins and/or whey proteins that are hydrolyzed with one or more proteolytic enzymes so as to reduce the risk of immunologic response, particularly adverse immunologic response, to the proteins, in a person predisposed to such response.

In one aspect hydrolyzates are provided comprising at least one milk protein hydrolyzed with at least one proteolytic enzyme that cleaves preferentially at a glutamic acid or aspartic acid residue and that leaves one or more immunological determinants present in the protein, the hydrolyzate comprising at least a beta lactoglobulin hydrolyzed in the region between amino acids 55-98, the hydrolyzate having reduced immunogenicity in a subject predisposed to having an immune reaction to the protein and wherein the hydrolyzate is enriched in at least the peptide AQSAPLRVYVE (SEQ ID No. 1), wherein at least one proteolytic enzyme is an endopeptidase and wherein the endopeptidase is not one having the amino acid sequence 
                5         10        15        20        25        30
  1 S V I G S D D R T R V T N T T A Y P Y R A I V H I S S S I G
 31 S C T G W M I G P K T V A T A G H C I Y D T S S G S F A G T
 61 A T V S P G R N G T S Y P Y G S V K S T R Y F I P S G W R S
 91 G N T N V D Y G A I E L S E P I G N T V G Y F G Y S Y T T S
121 S L V G T T V T I S G Y P G D K T A G T Q W Q H S G P I A I
151 S E T Y K L Q Y A M D T Y G G Q S G S P V F E Q S S S R T N
181 C S G P C S L A V H T N G V Y G G S S Y N R G T R I T K E V
211 F D N L T N W K N S A Q
 In one embodiment, the endopeptidase cleaves on the carboxy-terminal side of a glutamic acid residue or an aspartic acid residue. In one embodiment, the endopeptidase is from a *Streptomyces, Staphylococcus,* or a *Bacillus.*

In another embodiment of this aspect, the protein from which the hydrolyzate is produced is a whey protein, or comprises at least one of a lactalbumin or a lactoglobulin, such as an alpha lactalbumin or a beta lactoglobulin. In another aspect hydrolyzates are provided in accordance with claim 4.

The hydrolyzate is preferably enriched in peptides terminating in a glutamic acid or aspartic acid residue, as compared to the unhydrolyzed protein. In one embodiment, the hydrolyzate is enriched in one or more of the peptides ILLQKWE (SEQ ID NO: 2), KTKIPAVFKID (SEQ ID NO: 3), or KTKIPAVFKIDALNE (SEQ ID NO: 4). In other embodiments, it is enriched in one or more of the peptides ELEE (SEQ ID NO: 5), VATEE (SEQ ID NO: 6), PFTE (SEQ ID NO: 7), NSAEPE (SEQ ID NO: 8), VFGKE (SEQ ID NO: 9), AQSAPLRVYVE (SEQ ID NO: 1), ILLQKWE (SEQ ID NO: 2), KTKIPAVFKID
(SEQ ID NO: 3), KTKIPAVFKIDALNE (SEQ ID NO: 4), IQPTPE (SEQ ID NO: 10), LKPTPE (SEQ ID NO: 11), LKPTPEGD (SEQ ID NO: 12), LKPTPEGDLE (SEQ ID NO: 13), TKIPAVFKID (SEQ ID NO: 14), or TKIPAVFKIDALNE (SEQ ID NO: 15), and/or one or more of DQAME (SEQ ID NO: 16), GIHAQQKE (SEQ ID NO: 17), VLNE (SEQ ID NO: 18), VFGKE (SEQ ID NO: 19), RLHSMKE (SEQ ID NO: 20), DIKQME (SEQ ID NO: 21), KHPIKHQGLPQE (SEQ ID NO: 22), RPKHPIKHQGLPQE (SEQ ID NO: 23), PMIGVNQE (SEQ ID NO: 24), GIHAQQKEPMEGVNQE (SEQ ID NO: 25), RYLGYLE (SEQ ID NO: 26), LAYFYPE (SEQ ID NO: 27), FVAPFPE (SEQ ID NO: 28), KTTMPLW (SEQ ID NO: 29), LFRQFYQLD (SEQ ID NO: 30), FFVAPFPE (SEQ ID NO: 31), NLLRFFVAPFPE (SEQ ID NO: 32), or ENLLRFFVAPFPE (SEQ ID NO: 33).

Also provided herein are whey protein-containing hydrolyzates enriched AQSAPLRVYVE (SEQ ID NO: 1), preferably also enriched in one or more of the peptides: ELEE (SEQ ID NO: 5), VATEE (SEQ ID NO: 6), PFTE (SEQ ID NO: 7), NSAEPE (SEQ ID NO: 8), VFGKE (SEQ ID NO: 9), ILLQKWE (SEQ ID NO: 2), KTKIPAVFKID (SEQ ID NO: 3), KTKIPAVFKIDALNE (SEQ ID NO: 4), IQPTPE (SEQ ID NO: 10), LKPTPE (SEQ ID NO: 11), LKPTPEGD (SEQ ID NO: 12), LKPTPEGDLE (SEQ ID NO: 13), KTKIPAVFKID (SEQ ID NO: 14), or KTKIPAVFKIDALNE (SEQ ID NO: 15). The whey protein hydrolyzates have reduced immunogenicity in a subject predisposed to having an immune reaction to one or more whey proteins.

In one embodiment, the whey protein-containing hydrolyzate is preferentially enriched in one or more of the peptides ILLQKWE (SEQ ID NO: 2), KTKIPAVFKID (SEQ ID NO: 3), or TKIPAVFKIDALNE (SEQ ID NO: 4). The hydrolyzate of this aspect is produced through the use of an endopeptidase that preferentially cleaves on the carboxy-terminal side of a glutamic acid residue or an aspartic acid residue. The endopeptidase in one embodiment does not require a metal ion for its activity.

Also provided herein are food ingredients comprising a whey protein hydrolyzate enriched in AQSAPLRVYVE (SEQ ID NO: 1), preferably enriched in one or more of the peptides ILLQKWE (SEQ ID NO: 2), KTKIPAVFKID (SEQ ID NO: 3), or KTKIPAVFKIDALNE (SEQ ID NO: 4). The provided food ingredients have reduced immunogenicity in a subject predisposed to having an immune reaction to one or more whey proteins.

Provided herein also are manufactured food products comprising a whey protein hydrolyzate enriched in AQSAPLRVYVE (SEQ ID NO: 1), preferably enriched in one or more of the peptides ILLQKWE (SEQ ID NO: 2), KTKIPAVFKID (SEQ ID NO: 3), or KTKIPAVFKIDALNE (SEQ ID NO: 4). The manufacture food products have reduced immunogenicity in a subject predisposed to having an immune reaction to one or more whey proteins.

Examples of such manufactured food products provided in accordance herewith include infant formula, sports drinks, cheese-containing products, protein supplements, nutritional supplements, or meal replacement products.

Nonfood products that comprise any of the hydrolyzates described herein are also provided. In various embodiments, the nonfood product is a cosmetic, a lotion, or a cleanser for use on human skin. In one embodiment, the nonfood product comprises a whey protein hydrolyzate enriched in AQSAPLRVYVE (SEQ ID NO: 1), preferably enriched in one or more of the peptides ILLQKWE (SEQ ID NO: 2), KTKIPAVFKID (SEQ ID NO: 3), or KTKIPAVFKIDALNE (SEQ ID NO: 4), and has reduced immunogenicity in a subject predisposed to having an immune reaction to one or more whey proteins. A non-food product according to claim 23 is also provided.

Also provided are compositions comprising at least two hydrolyzates as described herein, wherein each hydrolyzate is produced with at least one different enzyme having a different specificity, or each hydrolyzate is produced with the same enzyme from a different protein or protein mixture. In another aspect is provided the use of claim 28.

The foregoing summary, as well as the following detailed description and examples are intended to illustrate various embodiments.

### BRIEF DESCRIPTION OF THE DRAWINGS

The appended figures serve to further explain or illustrate various aspects of the compositions or methods described more fully herein and exemplified below.
Figure 1: SDS-PAGE analysis showing time course of proteolysis of whey proteins over 20 hours of treatment with S-Glu or L-Glu. Lanes (left to right) 1, 2, 3: S-Glu treatment for 2, 4, and 20 hrs, respectively; Lane 4: Whey Control; Lanes 5, 6, 7: L-Endo treatment for 20, 4, and 2 hrs, respectively; Lane 8: Whey Control; Lanes 9-10: MW markers (standard proteins).
Figure 2: Representative mass spectrometric analysis of endopeptidase-digested whey protein. Sample was taken at 20 hr from sample digested with L-Glu. Panel A shows the detection of various MW species in the sample. Panel B shows the relative abundance of the prevalent degradation products.
Figure 3: Representative mass spectrometric analysis of endopeptidase-digested whey protein. Sample was taken at 20 hr from sample digested with S-Glu. Panel A shows the detection of various MW species in the sample. Panel B shows the relative abundance of the prevalent degradation products.
Figure 4: SDS-PAGE Analysis of Skim Milk-I, Hydrolysis by Endo Glu-Peptidase: Lanes (left to right): 1, 2, 3: S-Endo digestion at t=2, 4, and 20 hrs, respectively; Lane 5: Skim milk I control; Lanes 7, 8, 9: L-Endo digestion at t=2, 4, 20 hrs; Lane 10: MW Marker.
Figure 5: SDS-PAGE Analysis of Skim Milk-II Hydrolysis by Endo Glu-Peptidase: Lanes (left to right): 1, 2, 3: S-Endo digestion at t=2, 4, and 20 hrs, respectively; Lane 5: Skim milk II control; Lanes 7, 8, 9: L-Endo digestion at t=2, 4, 20 hrs; Lane 10: MW Marker.
Figure 6: SDS-PAGE Analysis of Casein Hydrolysis by Endo Glu: Lanes (left to right): 1, MW Marker; Lanes 2, 3, 4: S-Endo digestion at 2, 4, and 20 hrs respectively; Lane 5: Marker; Lanes 6, 7, 8: L-Endo digestion at 2, 4, and 20 hrs respectively; Lane 10: Sigma casein C-5890.

### DETAILED DESCRIPTION OF ILLUSTRATIVE EMBODIMENTS

### Acronyms & Abbreviations:

cP: centiPoise; dynamic viscosity units;
DFP: diisopropylfluorophosphate;
EDTA: ethylenediaminetetraacetic acid;
Endo-Glu: endopeptidase that cleaves next to a GLU residue in a polypeptide, preferably cleaving to the carboxy-terminal side of the GLU residue; also sometimes referred to herein as EndoGluC where the cleavage is definitively to the carboxy-terminal side of a GLU residue;
GMP(s): Good Manufacturing Practice(s);
HPLC: High performance liquid chromatography
hr(s): hour(s);
IU: International Units;
LC/MS: coupled liquid chromatography/mass spectrometry;
LC/MS/MS liquid chromatography coupled with dual mass spectrometry analysis;
L-Endo: A Glu-C endopeptidase from *Bacillus lichenformis;* also referred to sometimes herein as "L-Glu" or "L-MPR"
MS: Mass spectrometry, sometimes used generically to encompass coupled LC/MS and LC/MS/MS analyses;
MW: molecular weight;
PMSF: phenylmethylsulfonylfluoride;
S-Endo: Glu-C endopeptidase from *Bacillus subtilis;* also referred to sometimes herein as "S-Glu" or "S-MPR"
SDS PAGE: sodium dodecyl sulphate polyacrylamide gel electrophoresis;
Succ-AAPEpNA: succinyl-Ala-Ala-Pro-Glu-p-nitroanilide
t: time;
TFA: trifluoroacetic acid;
TP: total protein - e.g. total soluble protein in supernatant solution;
w/w: weight by weight (or weight to weight), usually describes a basis of determining a percentage; used *e.g.* for expressing concentrations of added components;
w/v: weight by volume (or weight to volume), an alternative basis for determining a percentage; used *e.g.* for expressing concentrations of added components;

### Definitions:

As used herein, "protein hydrolyzate" refers to the product resulting from the treatment of the protein with a proteolytic enzyme. The extent of proteolytic cleavage of the protein can range from minimal (e.g. cleavage of a single peptide bond on a single protein) to extensive (e.g. cleavage of all peptide bonds present within the sample that meet the specificity requirements of the proteolytic enzyme being used) depending on, for example, the conditions of the treatment, such as the length of the treatment, the temperature, the concentration of the protein, and the purity, concentration, and activity of the proteolytic enzyme.

As used herein, "milk protein" encompasses any naturally-occurring protein in the normal secretion of the mammary gland of a postpartum female mammal, or products derived therefrom, such as fractions thereof, or components made therefrom or thereof. The milk can be from any mammalian species including but not limited to cow, goat, sheep, buffalo, yak, camel, llama, alpaca, and human. Milk proteins from those mammals whose milk is used commercially or widely in various cultures and countries are preferred. It is to be noted that "milk protein" as used herein encompasses both the singular and the plural of the word "protein", thus, the term "milk protein" may refer to a single protein, or any mixture of one or more proteins, except as otherwise indicated.

"Whey protein" encompasses any protein found in any amount in "whey", the liquid by-product of cheese making that is separated from the curd. The whey resulting from the production of many cheeses is particularly low in micellar milk proteins, such as caseins, but relatively enriched in soluble proteins such as alpha lactalbumin and beta lactoglobulin. As with "milk protein" above, the term "whey protein" as used herein encompasses both the singular and the plural of the word "protein", thus, the term "whey protein" also may refer to a single protein, or any mixture of one or more whey proteins, except as otherwise indicated. It will be understood by the skilled artisan that whey proteins are in fact a subclass of milk proteins, and thus the term "milk protein" may include one or more whey proteins, except as otherwise indicated herein. Whey compositions may include, for example, milk, cream, and cheese whey. Whey derived from any cheese type may be used. Whey protein may be derived from any methods such as filtration, dialysis, evaporation, and reverse osmosis of cheese whey, or by any other process which results in the proteins typically described as "whey proteins".

As used herein, a "sensitive" individual is an individual predisposed to having an immune response or reaction to the protein in an unhydrolyzed form. Such immune response or reaction as a direct or indirect result of the consumption of, or exposure to, for example one or more milk proteins and/or whey proteins, is a measure of the immunogenicity of those proteins. Such proteins will demonstrate little to no immunogenicity in an individual who is not predisposed to having such an immune response to the protein, such an individual is sometimes referred to herein as "insensitive" or "not sensitive" to the one or more milk and/or whey proteins. Preferably, such an individual will not have a significant immune reaction (immunological response) to either the exposure to or consumption of the protein.

As used herein, "reduced immunogenicity" refers to any reduction, decrease, or amelioration of a measurable immunological response. The measurement of such response may be assessed *in vitro* or *in vivo.* For example the response may be measured directly or indirectly in a biological sample comprising tissue, cells, or fluid, or the like, or any combination thereof from an individual, or it may be assessed in the individual, either directly or indirectly. While in various embodiments provided herein, any mathematical decrease (or reduction) in such response, whether measured *in vitro* or *in vivo,* will suffice, it is preferred that the decrease be a more substantial one. Of course, the skilled artisan will appreciate that biological data such.as a measurement of an immunological response, are subject to potentially large variation within an individual, and from individual to individual. For example where the response is in a "sensitive" individual, the immune response is preferably substantially reduced (e.g. by at least about 50%, 60%, or 70%, or even about 80% or more). More preferably, only small or minimal differences are seen in such the sensitive individual with the protein hydrolyzates described herein, as compared to a measure of the immunological response from an individual who is not sensitive to one or more untreated milk or whey proteins. This is particularly preferable where the immunological response is deemed adverse, for example an allergic response. In such cases the decrease in the sensitive individual's immunological response (or measure thereof) may be at least about 85% to about 90%, more preferably about 90% to about 95%, or even more. In some case, a sensitive individual's response to the protein hydrolyzates described herein is not significantly different, statistically, from the response of an individual who is not sensitive. In yet other cases, the reduction in immunological response may be many-fold over that seen with the unhydrolyzed protein in a "sensitive" individual. For example, there may be about a 10-fold to 100-fold or even 1000-fold reduction in response. More preferably reductions of about 1000-fold to 10,000-fold or even 100,000-fold or greater reduction in a measurement of an immunological response from an individual consuming or exposed to the protein hydrolyzate compositions as disclosed herein, as compared to that individual's response to the unmodified proteins.

### Protein Hydrolyzates

In a first of its several aspects, protein hydrolyzates are provided comprising at least one milk protein hydrolyzed with at least one proteolytic enzyme that cleaves preferentially at a glutamic acid or aspartic acid residue and that leaves one or more immunological determinants present in the protein, the hydrolyzate comprising at least a beta lactoglobulin hydrolyzed in the region between amino acids 55-98, the hydrolyzate having reduced immunogenicity in a subject predisposed to having an immune reaction to the protein and wherein the hydrolyzate is enriched in at least the peptide AQSAPLRVYVE (SEQ ID No. 1), wherein at least one proteolytic enzyme is an endopeptidase and wherein the endopeptidase is not one having the amino acid sequence 
                    5                   10                  15                  20                  25                  30
  1 S V I G S D D R T R V T N T T A Y P Y R A I V H I S S S I G
 31 S C T G W M I G P K T V A T A G H C I Y D T S S G S F A G T
 61 A T V S P G R N G T S Y P Y G S V K S T R Y F I P S G W R S
 91 G N T N Y D Y G A I E L S E P I G N T V G Y F G Y S Y T T S
121 S L V G T T V T I S G Y P G D K T A G T Q W Q H S G P I A I
151 S E T Y K L Q Y A M D T Y G G Q S G S P V F E Q S S S R T N
181 C S G P C S L A V H T N G V Y G G S S Y N R G T R I T K E V
211 F D N L T N W K N S A Q
 In one embodiment, the hydrolyzate has reduced immunogenicity in a subject predisposed to having an immune reaction to the protein in an unhydrolyzed form.

In certain embodiments, the hydrolyzate containing the at least one milk protein is hydrolyzed with an endopeptidase. The specificity of the endopeptidase is preferably limited. As the skilled artisan will appreciate, for use in food products, extensive, nonspecific proteolysis of proteins can lead to undesirable properties, such as off-flavors and/or loss of functionality. The endopeptidase for use herein thus preferentially cleaves to only a limited extent, for example by having specificity for one or more particular amino acids, or particular types of amino acids on either or both of the carboxy-terminal and amino-terminal side of the cleavage site.

In certain presently preferred embodiments, the endopeptidase has a specificity for the carboxy-terminal side of hydrophilic amino acid residues, particularly glutamic acid or aspartic acid residues. In one embodiment, the endopeptidase specifically cleaves on the carboxy-terminal side of glutamic acid residues and/or aspartic acid residues. In another embodiment, the endopeptidase cleaves exclusively or almost exclusively at the carboxy-terminal side of glutamic acid residues.

In certain embodiments, the endopeptidase is in the serine protease family. It is inhibited, at least partially, if not fully, by phenylmethylsulfonylfluoride or diisopropylfluorophosphate. In certain embodiments, the endopeptidase is from a *Streptomyces, Staphylococcus,* or a *Bacillus* spp. Alternately, the endopeptidase can be from any source that is suitable for use in food or feed production. In one embodiment, the endopeptidase is from *Bacillus subtilis* or from *Bacillus lichenformis.* In certain embodiments, the endopeptidase is a 23.6 kDa protein from *B. lichenformis,* the amino acid sequence of which is provided in the European Molecular Biology Laboratory (EMBL) database at the accession number EMBL P80057. Alternatively, the endopeptidase is a 23.9 kDa protein from *B. subtilis,* the amino acid sequence of which is provided in the EMBL database at accession number EMBL P39790. In yet another embodiment, the enzyme is any endopeptidase which meets the above-stated criteria, with the proviso that it is not an endopeptidase disclosed in US Patent No. 5,866,357.

The source of milk for the protein is not limited to any particular mammalian species, and thus it is contemplated that milk from species including but not limited to cow, goat, sheep, buffalo, horse, camel, yak, llama, alpaca, and humans may be used herein. In one embodiment, the milk proteins originate from a species that is different from the individual that is exposed to or to consume the milk protein. In another embodiment, an individual is sensitive to milk proteins from its own species. Thus, the milk proteins may be from the same species as the individual. In such embodiments, the modified proteins reduce the immunogenicity of the milk protein in that individual. In one embodiment, the individual (sometimes referred to herein as a "subject") is human, and the milk is from a nonhuman animal species whose milk is frequently consumed by humans, for example, cow, goat, sheep, or buffalo.

In certain embodiments, the at least one milk protein is a soluble, non-micellar protein. The milk protein thus is preferably not a casein in such embodiments. In one embodiment, the milk protein is soluble protein, for example one or more of the proteins typically found in whey resulting from the production of any cheese produced from curd.

In a particular embodiment, the protein includes at least one of a lactalbumin or a lactoglobulin. As the skilled artisan will appreciate, there are a number of such proteins that have been identified in the milk from various mammals. Among the prevalent whey proteins are alpha lactalbumin and beta lactoglobulin, both of which are suitable for use herein. It is to be understood that a whey protein will not typically be isolated from other whey proteins prior to use herein. The use of such isolated or even purified milk proteins is nonetheless also contemplated herein.

Because of the potential immunogenicity of such milk proteins, as discussed above, it is desirable for the endopeptidase to cleave peptides that may serve as immunological determinants (e.g. antigenic determinants) that may stimulate, provoke, induce, or exacerbate an immune response in an individual. Such immune responses include, but are not limited to those primarily under control of the T-lymphocytes and those more frequently associated with B-lymphocytes of the immune system. They also include such responses, wherein the antibodies produced are of any type. The skilled artisan will appreciate that among the many measurable responses, frequently Ig-E antibodies are produced in response to such proteins in a responsive individual.

For the purposes herein a "reduction" of immunogenicity means at least a mathematical decrease in the measure of the immune response, whether *in vivo* or *in vitro.* A statistically significant decreased is preferred, where such analysis is possible and appropriate. The artisan skilled in statistical analysis will appreciate that the determination of a statistical difference can be performed using, for example, Student's T-test, or any other analysis appropriate to the nature and design of the data gathering methodology.

Thus, to help minimize the potential immunogenicity of the milk proteins, such as the whey proteins, alpha lactalbumin and beta lactoglobulin, the endopeptidase preferably cleaves one or more immunological determinants present in the protein, for example, the region in beta lactoglobulin between amino acids 55-98. Such cleavage results in the hydrolyzate becoming enriched, relative to the unhydrolyzed protein in one or more of certain peptides. In one embodiment, the hydrolyzate is enriched in AQSAPLRVYVE (SEQ ID NO: 1), preferably in one or more of the peptides: ILLQKWE (SEQ ID NO:2), KTKIPAVFKID (SEQ ID NO:3) ,and KTKIPAVFKIDALNE (SEQ ID NO:4).

In certain embodiments, the hydrolyzate is generally enriched in peptides terminating in a glutamic acid or aspartic acid residue, as compared to the unhydrolyzed protein. When the milk protein includes a whey protein, in one embodiment the hydrolyzate is enriched in AQSAPLRVYVE (SEQ ID NO:1), preferably enriched in one or more of the peptides: ELEE (SEQ ID NO:5), VATEE (SEQ ID NO:6), PFTE (SEQ ID NO:7), NSAEPE (SEQ ID NO:8), VFGKE (SEQ ID NO:9), ILLQKWE (SEQ ID NO:2), KTKIPAVFKID (SEQ ID NO:3), TKIPAVFKIDALNE (SEQ ID NO:4), IQPTPE (SEQ ID NO: 10), LKPTPE (SEQ ID NO: 11), LKPTPEGD (SEQ ID NO:12), LKPTPEGDLE (SEQ ID NO: 13), TKIPAVFKID (SEQ ID NO: 14), and TKIPAVFKIDALNE (SEQ ID NO: 15).

Alternatively, in an embodiment where the milk protein includes a casein, the hydrolyzate may be enriched in one or more of the peptides: DQAME (SEQ ID NO:16), GIHAQQKE (SEQ ID NO: 17), VLNE (SEQ ID NO: 18), VFGKE (SEQ ID NO: 19), RLHSMKE (SEQ ID NO:20), DIKQME (SEQ ID NO:21), KHPIKHQGLPQE (SEQ ID NO:22), RPKHPIKHQGLPQE (SEQ ID NO:23), PMIGVNQE (SEQ ID NO:24), GIHAQQKEPMEGVNQE (SEQ ID NO:25), RYLGYLE (SEQ ID NO:26), LAYFYPE (SEQ ID NO:27), FVAPFPE (SEQ ID NO:28), KTTMPLW (SEQ ID NO:29), LFRQFYQLD (SEQ ID NO:30), FFVAPFPE (SEQ ID NO:31), NLLRFFVAPFPE (SEQ ID NO:32), and ENLLRFFVAPFPE (SEQ ID NO:33).

The skilled artisan will note that nothing herein precludes the use of both casein and whey proteins as a source for the hydrolyzate, in which cases, the hydrolyzate may be enriched for any or all of the foregoing peptides (SEQ ID NOs: 1-32), or any combinations thereof.

### Whey Protein-Containing Hydrolyzates

Also provided herein are whey protein-containing hydrolyzates enriched in AQSAPLRVYVE (SEQ ID NO:1). The whey protein hydrolyzates are enriched in one or more of the peptides: ELEE (SEQ ID NO:5), VATEE (SEQ ID NO:6), PFTE (SEQ ID NO:7), NSAEPE (SEQ ID NO:8), VFGKE (SEQ ID NO:9), ILLQKWE (SEQ ID NO:2), KTKIPAVFKID (SEQ ID NO:3), KTKIPAVFKIDALNE (SEQ ID NO:4), IQPTPE (SEQ ID NO: 10), LKPTPE (SEQ ID NO:11), LKPTPEGD (SEQ ID NO:12), LKPTPEGDLE (SEQ ID NO: 13), TKIPAVFKID (SEQ ID NO:14), and TKIPAVFKIDALNE (SEQ ID NO:15), and have reduced immunogenicity in a subject predisposed to having an immune reaction to one or more whey proteins.

As discussed above, the immune reaction can encompass any of the reactions of an individual to a foreign protein. The measure of such a reaction can encompass any of the techniques, *in vitro*, *in vivo*, clinical or otherwise, known to the person of skill in that art. As above, a "reduction" means at least a mathematical decrease. A statistically significant decrease is preferred, where such analysis is possible and appropriate. In a one embodiment, the subject is a human with allergies to whey proteins from cow's milk. The hydrolyzate provides at least about a 50% decrease in the measure of immunogenicity as compared to the unhydrolyzed whey protein. In other embodiments, there is about a 70%, 80%, or 90% or more reduction in the measure of immunogenicity. In yet other embodiments, there is about a 10-fold to 100-fold reduction in the immunogenicity, a 100-fold to 1000-fold reduction, a 1000-fold to 10,000-fold reduction, or even more. In one embodiment, the hydrolyzed protein composition results in reduced immunogenicity in the sensitive subject such that there is almost no detectable differences between the immunological response to the hydrolyzed protein in a sensitive subject, and the response in one who is not sensitive. Preferably, there are no statistical differences in the immunological responses between a sensitive and non-sensitive individual upon exposure to, or consumption of, the whey protein-containing hydrolyzate.

In certain embodiments, the whey protein-containing hydrolyzate enriched in AQSAPLRVYVE (SEQ ID NO:1), is enriched, relative to a comparable but untreated whey protein sample, in one or more of the peptides: ILLQKWE (SEQ ID NO: 2), KTKIPAVFKID (SEQ ID NO: 3), and KTKIPAVFKIDALNE (SEQ ID NO: 4).

The whey protein-containing hydrolyzate can be produced through the use of one or more endopeptidases that preferentially cleave on the carboxy-terminal side of glutamic acid residues and/or aspartic acid residues. In a particular embodiment, the whey protein-containing hydrolyzate is produced through the use of an endopeptidase that does not use or require a metal ion for its activity, and thus is not a strict metalloproteinase. The skilled artisan will appreciate that the use of metal chelators such as EDTA can help to determine the absence of a metal requirement for any enzyme, e.g. a protease or a peptidase.

### Uses of the Protein Hydrolyzates:

In another aspect, food additives, supplements, and ingredients comprising a whey protein-containing hydrolyzate or a milk protein hydrolyzate, enriched in AQSAPLRVYVE (SEQ ID NO: 1), as described above, are provided.

Hydrolyzates, preferably whey hydrolyzates, enriched in one or more of the peptides: ILLQKWE (SEQ ID NO: 2), KTKIPAVFKID (SEQ ID NO: 3), and KTKIPAVFKIDALNE (SEQ ID NO: 4) relative to the unhydrolyzed protein are preferred for such uses.

The additives have reduced immunogenicity in a subject predisposed to having an immune reaction to one or more milk or whey proteins.

Also provided herein are manufactured food products comprising a milk or whey protein hydrolyzate enriched in AQSAPLRVYVE (SEQ ID NO:1), as described herein. Preferably the hydrolyzates are enriched in one or more of the peptides: ILLQKWE (SEQ ID NO: 2), KTKIPAVFKID (SEQ ID NO: 3), and KTKIPAVFKIDALNE (SEQ ID NO: 4), and have reduced immunogenicity in a subject predisposed to having an immune reaction to one or more milk whey proteins.

In certain embodiments, the manufactured food product is an infant formula, a sports drink, a cheese-containing product, a protein supplement, a nutritional supplement, or a meal replacement product. In preferred embodiments, the hydrolyzate is a whey protein hydrolyzate and the subject has an allergic reaction to unhydrolyzed lactoglobulin and/or lactalbumin.

It will be apparent to the skilled artisan that the compositions provided herein may also be used in connection with cheese making. The use of the compositions is particularly well-suited to the production of various hard and soft cheeses. The compositions provided herein can be used to reduce immunogenicity of such products. The cheese can be made entirely with milk that is hydrolyzed as provided herein, or prior to curding, a milk or whey protein hydrolyzate compositions can be added to the milk being used for cheese production. Such uses will provide cheese products with superior qualities in terms of nutritional content and functional properties.

Similarly, the milk and whey hydrolyzate compositions provided herein are useful for the manufacture of other dairy products including but not limited yogurt, cottage cheese, or sour cream production. The skilled artisan will also appreciated that the hydrolyzates are adaptable for spray drying, concentrating, or other further processing steps. Spray dried hydrolyzates are particularly well-suited for use for applications where whey solid might currently be used. They can also be used as a milk solids replacement where achieving reduced immunogenicity is desirable.

In certain embodiments, the milk and whey hydrolyzate compositions provided herein are used in other food products which frequently contain one or more milk products or byproducts that are not declared on the label, or which may not have a label, as such, provided to the consumer. In some cases, even though the ingredients are listed on a label, consumers who are not thoroughly educated in nutrition do not understand that certain of the listed ingredients actually are milk or whey protein containing ingredients. Such products pose a real risk to sensitive individuals, for example those who may suffer severe allergic reactions, and may in fact be life-threatening to those individuals. The compositions provided herein are readily adapted for such uses and can be used for example in beverages, such as coffee, tea, or modifications and derivatives thereof, sports drinks, and the like, as well as puddings, fillings, and snacks. Many other products have minor amounts of milk or whey ingredients added to provide known beneficial functionality for processing or consumer acceptance. The hydrolyzates described herein can readily be substituted for the ingredients from which they were derived. They preferably retain the functional properties, such as texture, aroma, mouthfeel, dispersability, solubility, and the like, of the ingredients from which they were derived. They also provide improved flavor (e.g. reduced bitterness) and preferred foaming properties relative to alternatives such as whey proteins that are more fully hydrolyzed with less specific enzymes. In some embodiments, the hydrolyzed proteins have improved gelling, water holding, or water binding capacity compared to the unhydrolyzed proteins.

In another embodiment the hydrolyzed whey or milk proteins described herein are used in the manufacture of a topically applied product, such as a lotion, cream, ointment, rub, cleanser, or the like. Accordingly, such products comprising the hydrolyzed protein compositions described herein are herein contemplated. Such products are useful for example for therapeutic purposes, for example, to provide relief from dry skin, itching, discomfort, and the like. These products preferably comprise, in addition to the hydrolyzed protein component, a lipid, wax, oil, water in oil emulsion, oil-in-water emulsion, or the like as a base,. Typically, they may further comprise one or more fragrance components, as well as other ingredients such as surfactants or emulsifiers. Cosmetic products and other appearance aids or beauty aids comprising the milk or whey protein hydrolyzates described herein are also provided. In one embodiment, the cosmetic product is applied to the face, cheeks, lips, or eyes of a person. In another embodiment the product is used anywhere on the body to help improve the cosmetic appearance of the skin or, for example, to diminish the appearance of wrinkles moles, freckles, scars, blemishes, and the like.

### Methods of Making:

The hydrolyzed milk and or whey protein compositions described above may be made by the addition of one or more proteolytic enzymes to a milk protein- or whey protein-containing liquid or semisolid.

The enzymatic treatment may be conducted by introducing or dispersing the protease, or a combination of appropriate proteases, such as one or more endoproteases or endopeptidases into a milk or whey composition, or into a composition containing milk and/or whey proteins as described herein to bring the enzyme in contact with the protein substrate. Introducing the enzyme into the protein composition is followed by allowing the enzyme reaction to take place by holding the mixture under conditions suitable therefore - e.g. for an appropriate holding time, at an appropriate temperature and pH, and in the presence of any required cofactors. Treatment of the milk or whey protein compositions with the enzyme(s) described above, may be carried out under conditions chosen to suit the selected enzyme(s) according to principles well known in the art. The skilled artisan will appreciate that where the desired end-product is for human consumption, the conditions may require that proper sanitation and good manufacturing practices (GMPs) be followed during the holding and treatment of the protein-containing material being used. Similarly, products intended for use other than consumption, such as topically-applied lotions or cosmetic products may have regulatory requirements associated with their manufacture.

Notwithstanding the foregoing, the enzymatic treatment may be conducted at any suitable pH, such as about pH 2 to 10, pH 4 to 9, or pH 5 to 7. It may be preferred to use a pH of about 7 to 8 for some of the enzymes discussed herein. The skilled artisan will appreciate that each enzyme will have a particular performance over a pH range, and that it may not be always necessary or even desirable to use an enzyme at a pH where its activity is highest. In similar fashion, the skilled artisan can readily select a suitable temperature, or temperature for the enzymatic treatment step. Factors affecting the temperature selection include but are not limited to the thermostability of the enzyme or enzymes being used, the thermostability of the proteins being hydrolyzed and the compositions comprising said proteins, as well as changes in the relative activity of the enzyme with temperature.

Optionally, after a suitable time for the enzymatic treatment, e.g. after a certain amount of proteolysis has taken place, for example, a predetermined desirable amount of proteolysis based on considerations such as functional and immunogenic properties, the enzyme may be removed or reduced in activity. The enzyme may be partially or completely inactivated through processes known in the art for removal or inactivation of enzymes used in food or feed processing. In one embodiment, the enzyme is thermally inactivated when the proteolysis is sufficiently extensive to provide the reduced immunogenic properties described herein. In another embodiment, the extent of proteolysis is monitored with a rapid measurement thereof, for example, % total soluble protein, viscosity. The enzyme is inactivated when the rapid measurement reaches the predetermined desirable reading. Preferably the enzyme activity is thermally inactivated, more preferably the thermal inactivation is combined with a processing step such as a required heating process, pasteurization, or homogenization.

Suitable enzyme dosages will usually be in the range of about 0.01-1% (w/w). In various embodiments, the milk and or whey proteins will be present at, for example, about 1-60%, 5-50%, 20-40%, 10-45%, or about 10-15% milk whey protein content. Thus, enzyme dosage might include amounts such as, e.g., 0.1-1.0%, particularly 0.2% (w/w), corresponding to 2000 IU per 100 g of milk or whey protein.

One IU (International Unit) is defined as the amount of enzyme producing one micromole of protein hydrolysis product per minute under standard conditions. "Standard conditions" for the purpose of determining IU are the use of 10-15% whey protein solids in water, or skim milk, pH 7, 40 °C temperature. In one embodiment, the enzyme dosage is based on w/w protein content of the composition being treated, as illustrated in the examples.

Alternatively, the enzyme dosage may be determined in other ways, such as by the use of other assays described herein. Such assays can all provide indicators of the extent (or degree) of protein hydrolysis and include determinations of % hydrolysis, viscosity measurements, gel electrophoresis, and mass spectrometry. In other words, an amount of enzyme required to produce the desired or predetermined result in a given amount of incubation time can also be calculated empirically, or based on other data. In one embodiment, an amount of enzyme can be selected to provide a specific drop in viscosity; in another an amount of enzyme to produce certain digestion fragments as determined by gel electrophoresis or mass spectrometry (MS) analysis.

In one embodiment, the enzyme dosage may be back-calculated by the degree of hydrolysis of a protein sample. The "degree of hydrolysis" is a measure of the number of peptide bonds cleaved by a proteolytic enzyme. In this case, the maximum degree of hydrolysis would result from cleavage at substantially all glutamate and possibly aspartate residues in the whey protein molecules. The degree of hydrolysis may be determined by art-known methods, for example by using a TNBS (trinitrobenzene sulphonic acid) assay as described in J. Adler-Nissen, J. Agric. Food Chem. 27: 1256 (1979).

Other methods of determining dose of enzyme or enzyme activity assays, such as through the detectable substrates, such as colorimetric or artificial substrates. Suc-AAPEpNA (succinyl-Ala-Ala-Pro-Glu-p-nitroanilide) has been used. Conversion to concentration of active EndoGluC protease can be made by obtaining an accurate specific activity rate measurement and converting Units of activity per unit volume to amount of enzyme using a standard curve or predetermined conversion factor. For example, for certain experiments used herein, the conversion factor for L-MPR was 0.348 U/mg of enzyme, and that for S-MPR was 0.549 U/mg. The skilled artisan will appreciate how to determine the dosage or activity required in accordance with the foregoing.

The enzymatic treatment may be conducted in batchwise operations, e.g. in a tank with stirring. The treatment may also be continuous, such as in a series of stirred tank reactors. The treatment may also encompass the use of immobilized enzymes and various modified enzymes, provided that an appropriate specificity of hydrolysis is retained by the immobilized or modified enzyme activity.

### Examples

### Example 1

### Digestion of Milk Proteins with Endopeptidase of limited specificity:

Enzymes: *Bacillus licheniformis* "Endo-Glu" peptidase ("L-Glu"): The enzyme ("L-MPR" or "Endo-GluC") from *B. licheniformis* was expressed in *B. subtilis* and purified from 2-14L fermentations, pooled and concentrated. The concentration of purified enzyme (>90% purity) was 25.1 mg/mL. This 23.6 kDA protein has sequence similarity to V8 protease from *Streptomyces griseus* and other Glu/ASP specific proteases (*e.g.* with specificity for Glu-Xaa, ASP-Xaa at the cleavage site). It is a serine endopeptidase that is partially inhibited by EDTA, however it is not a metalloprotease. It exhibits a pH optimum around 7.5-8. Higher ion strength lowers the activity. The sequence is known and can be found in public databases. For example, it is provided at UniProtKB/Swiss-Prot Primary Accession Number P80057, available from the European Molecular Biology Laboratory database available on the worldwide web. *See* "Purification, characterization, cloning, and expression of a glutamic acid-specific protease from Bacillus licheniformis ATCC 14580" J. Biol. Chem. 267: 23782-23788 (1992).

*Bacillus subtilis* "Endo-Glu" peptidase ("S-Glu"): The enzyme ("S-MPR" or "Endo-GluC") from *B. subtilis* was expressed in *B. subtilis* and purified from 2-14 L fermentations, pooled and concentrated. The concentration of purified enzyme (>85% purity) was 3.5 mg/mL. A 23.9 kDa serine protease, the protein has sequence similarity to that of the *B. licheniformis* Endo-Glu peptidase "L-Glu". The S-Glu protease is relatively stable over a wider range of pH. It is not as specific as the L-Glu enzyme from *B. licheniformis.* The sequence is known and can be found in public databases. For example, it is provided at UniProtKB/Swiss-Prot Primary Accession Number P39790, available from the European Molecular Biology Laboratory database available on the worldwide web. *See* "Gene encoding a glu-specific extracellular endopeptidase in Bacillus subtilis."; J. Bacteriol. 172: 1024-1029 (1990)., and "Purification and characterization of a glutamic acid-specific endopeptidase from Bacillus subtilis ATCC 6051: application to the recovery of bioactive peptides from fusion proteins by sequence-specific digestion." Appl. Microbiol. Biotech. 48: 27-33 (1997).

### Methods:

To assess the time course of proteolytic digestion, 12% whey protein (13.3 gram of whey powder (Lacprodan DI-9224 (Arla Foods)) in 100 ml water) was dissolved in reagent-grade water. Fifteen (15) g of this solution each was placed into each of four 50 ml tubes and incubated to reach at 40 °C in a shaker-incubator (e.g. for fifteen minutes). Enzyme purified as described above was added to each of three of the 50 ml tubes. 500 µl of either S-MPR at a concentration of 1.5 mg/ml, or L-MPR endoprotease at a concentration 4.2 mg/ml, were used. Activity was measured using Succ-AAPEpNA as substrate. The enzymes were greater than about 98% homogenous by SDS-PAGE. The fourth tube was a control tube which did not receive added enzyme. The tubes were incubated at 40 °C, 175 rpm in an orbital shaker for the indicated time. Tubes were removed at the end of their incubation time (2, 4, and 20 hours, respectively), and centrifuged at 3600 rpm (about 2800 x g) for ten minutes to separate precipitate and supernatant fluid.

Supernatant fluid was tested for total protein (TP) remaining soluble in the supernatant (g/l), % dry solid, and viscosity measurements. The supernatants were also analyzed electrophoretically using SDS-PAGE, to monitor the hydrolysis. Mass spectrometric (MS) analyses was performed to confirm the presence of individual proteolytic cleavage products.

### Mass Spectrometric Analysis:

*Sample Preparation:* Hydrolyzed protein samples were prepared as described. Approximately 20 µl of each digested protein samples were analyzed by a high-performance liquid chromatography coupled to a LCQ Ion Trap Mass Spectrometer system (LC/MS).

*LC*/*MS*/*MS Analysis:* All MS and MS/MS data were acquired using the Surveyor HPLC system coupled to the LCQ Advantage Ion Trap MS (ThermoFinnigan, San Jose, CA). A Vydac reverse phase C 18 column (2.1 X 150 mm) was used for all proteolytic digested samples using the HPLC gradient from 0% to 70% Solvent B over 65 minutes at the flow rate of 200 µl/min. Solvent A: 0.1% TFA in water and Solvent B: 0.08% TFA in acetonitrile. Data Processing was performed using the TurboSEQUEST and the Xcalibur programs (ThermoFinnigan).

The results from the MS analysis were then used with the Mascot program, which allows peptide digest fragments to be used as the only data from which the source protein and its primary sequence can be identified using the public databases such as that maintained by the National Center for Biotechnology Information. More information on Mascot, and its predecessor, Mowse, can be found on the world wide web, for example at the web site (www.matrixscience.com) maintained by Matrix Science Inc., (Boston, MA).

### Results:

The endopeptidase S-Glu at a final enzyme protein concentration of 0.188% to whey substrate protein concentration of 12%, resulted in a 5.5% decrease in total protein (TP) within 2 hours. Over the next 4 to 20 hours, TP decreased further, to a maximum decrease of about 20%.

Precipitated curd had a hold-up water volume (i.e., a measure of curd water retention) of about equal weight of water to curd.

The viscosity of the supernatant improved from 3.05 cP to 5.99 cP after 20 hour incubation with S-Glu enzyme.

Similar results were observed with L-Glu enzyme at the same final concentration as above. Within 2 hours, a 6.9 % decrease of TP was observed, improving to 10% by the 4 hour time point. The decrease reached 14% by the end of the 20 hour incubation.

Precipitated curd had a hold up water volume of about 70% weight of the weight of the protein.

The viscosity of the supernatant remained about the same during the course of the 20 hour incubation with L-Glu endopeptidase.

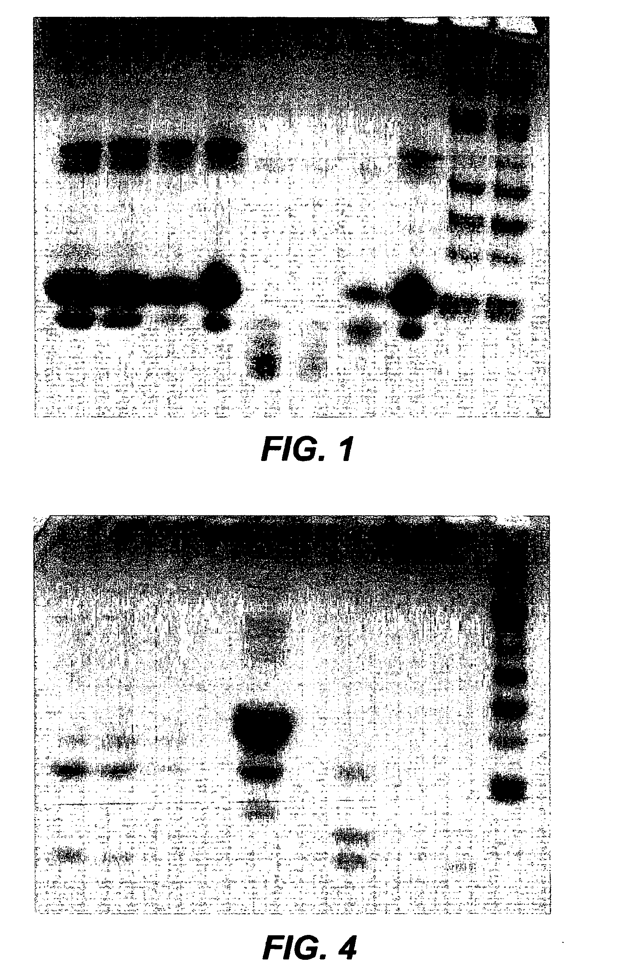

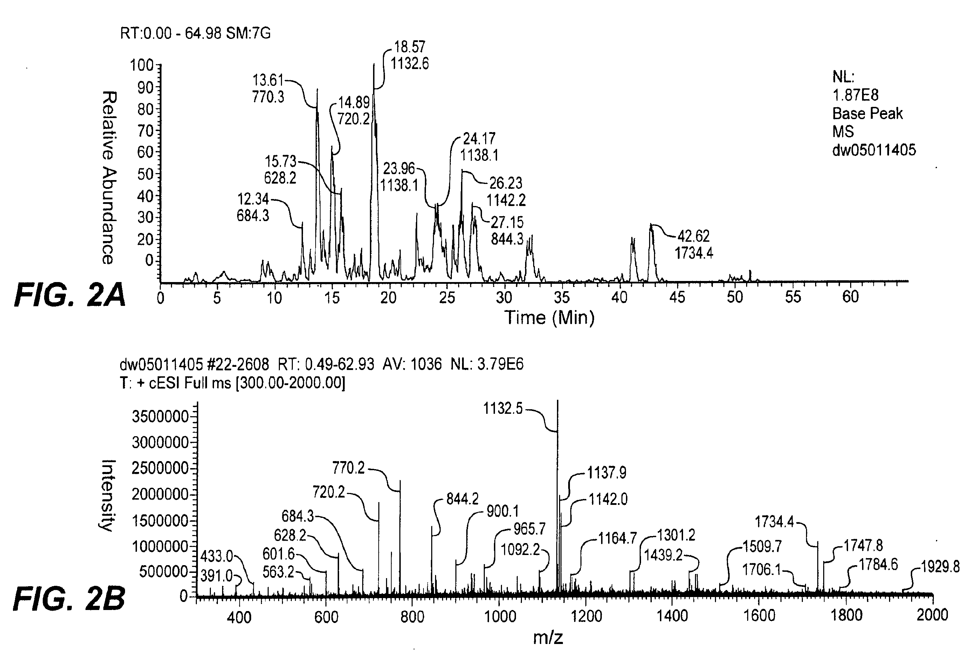

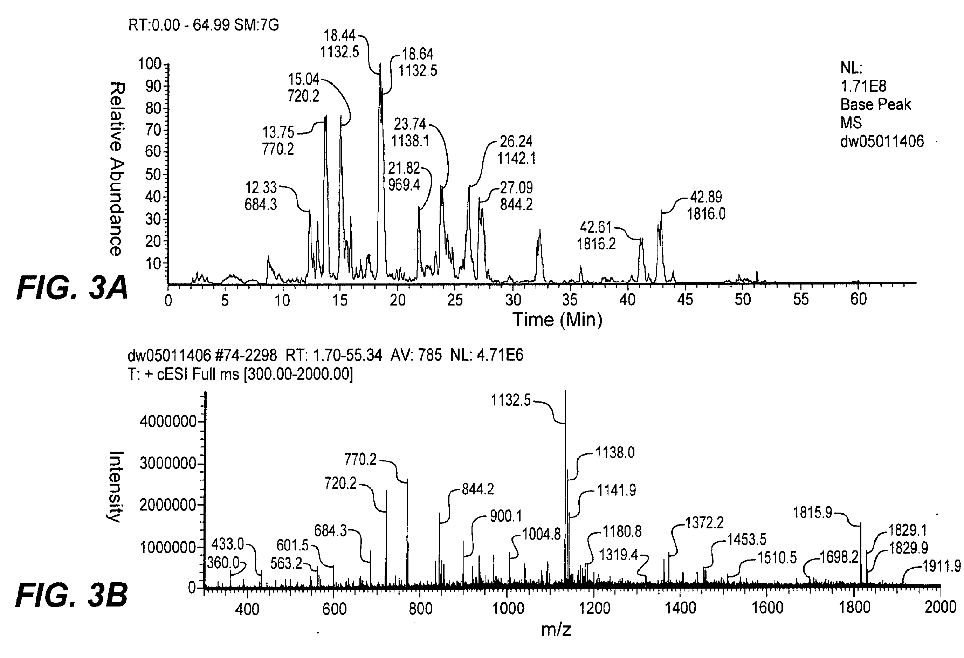

The results of the time course as revealed by SDS-PAGE are shown in Figure 1 for both the S-Glu and L-Glu enzyme treatments. The results of the mass spectrometric analysis of the 20 hr digestion products of the whey sample are shown in Figure 2 for L-Glu, and Figure 3 for S-Glu.

The whey protein endopeptidase hydrolyzate samples were enriched in specific peptides the identity and relative amounts of which were verified using the LC/MS/MS detailed above.

Tables 1 (a) and (b) and 2 (a) and (b) below show the data obtained from the Mascot analysis of the peptide data for the whey samples digested with L-Glu and S-Glu, respectively, at 20 hr of digestion.

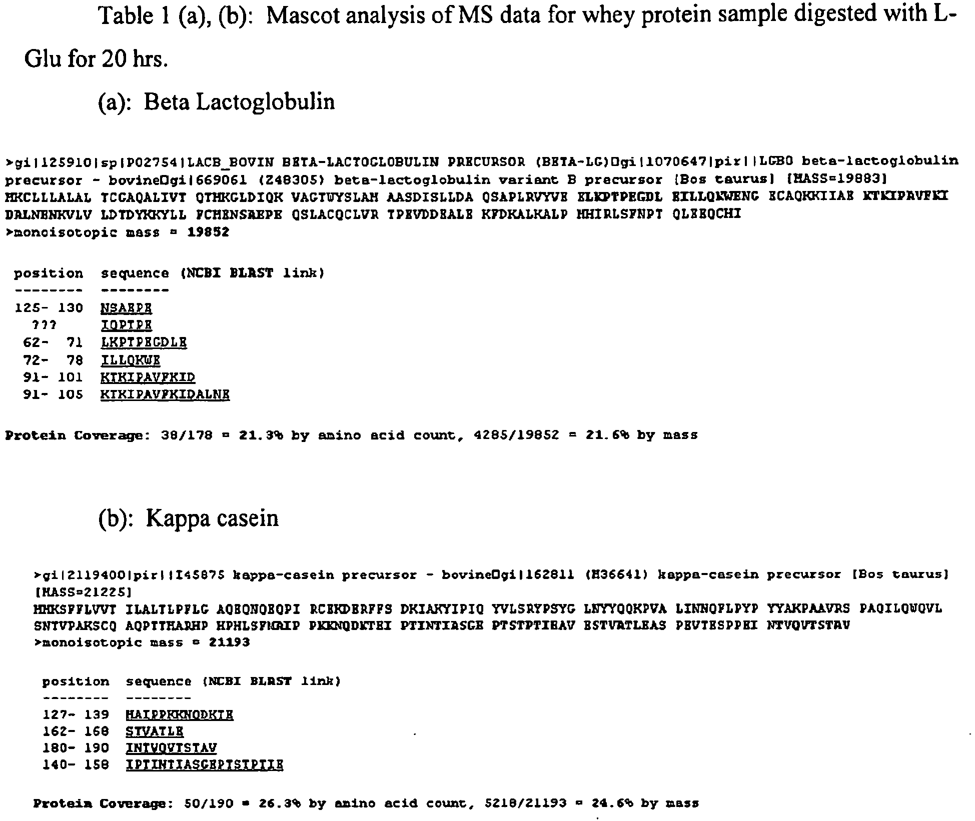

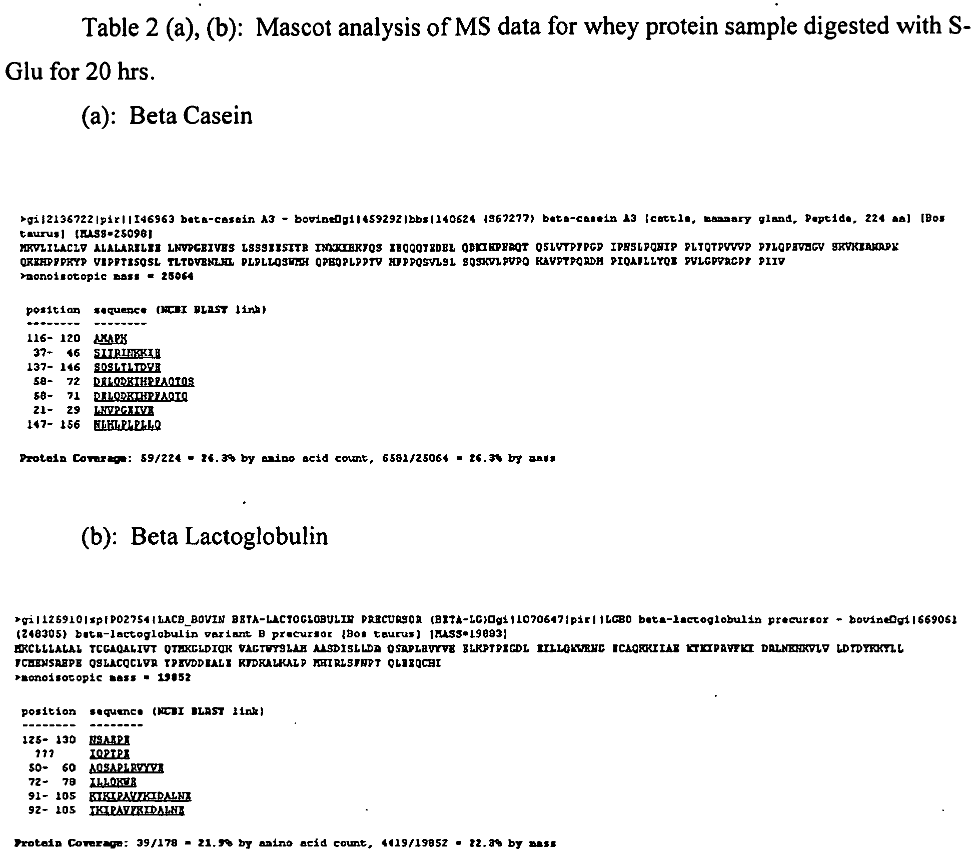

### Example 2

### Digestion of milk proteins using the endopeptidases, S-Glu and L-Glu

Methods: Ten (10) g of skim milk (two sources, Berkeley Farms and Sunnyside) were placed in each of eight 15 ml tubes. The tubes were warmed up to 40 °C for 15 minutes in an incubator-shaker. The enzyme (S-Glu or L-Glu) was added (500 µl of a 3.8 mg/ml stock solution) to each of three tubes for each enzyme. One tube served as a control for each source of skim milk and did not receive any added enzyme. The incubation continued for the indicated time under the foregoing conditions. One tube for each enzyme treatment was removed at 2, 4, 20 hr. The tubes were then centrifuged to separate the precipitate and supernatant. Supernatants were tested for TP (total protein) (g/l), % dry solid, and viscosity measurements. SDS-PAGE analysis was used to monitor the endopeptidase cleavage. Mass Spectrometric analysis was done as detailed above to confirm the amount and identity of the cleavage products generated. Mascot analysis using the peptides generated was also performed using the mass spectrometry data collected.

**Results:** Two samples of skim milk treated with S-Glu at final concentration of 0.19 mg/g of skim milk yielded a heavy coarse curd within 2 hour of treatment. About 43%-57% of total protein of skim milk was precipitated within two hours of enzyme treatment. The precipitation was almost complete, as no further significant decrease of total protein in the supernatant was observed at 4 or 20 hr for either of the two skim milk sources.

In contrast with S-Glu, samples treated with L-Glu showed improved precipitation of the protein from skim milk over time. For skim milk sample I, L-Glu endopeptidase treatment caused a decrease of 32% in protein concentration at t = 2hr, 42% by 4 hr, and 62% by 20 hours. In skim milk sample II treated with L-Glu, TP concentration decreased by 11.5% at t= 2 hr, 41% by 4 hours, and 51.7% at 20 hours.

As can be seen from the SDS-PAGE results (Figures 3 and 4, skim milk I and II, respectively), both of the endopeptidases L-Glu and S-Glu were able to cause casein to precipitate within 2 hrs from skim milk and had significant hydrolysis of casein, as well as lactoglobulin and lactalbumin.

The endopeptidase hydrolyzed skim milk samples were enriched in specific peptides the identity and relative amounts of which were verified using the LC/MS/MS detailed above. Tables 3 (a), (b), and (c) below show the data obtained from the Mascot analysis of the peptide data for skim milk I sample digested with L-Glu after 20 hr of digestion.

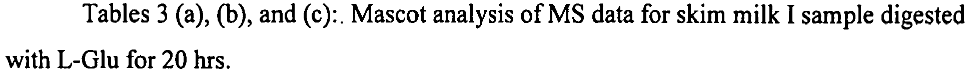

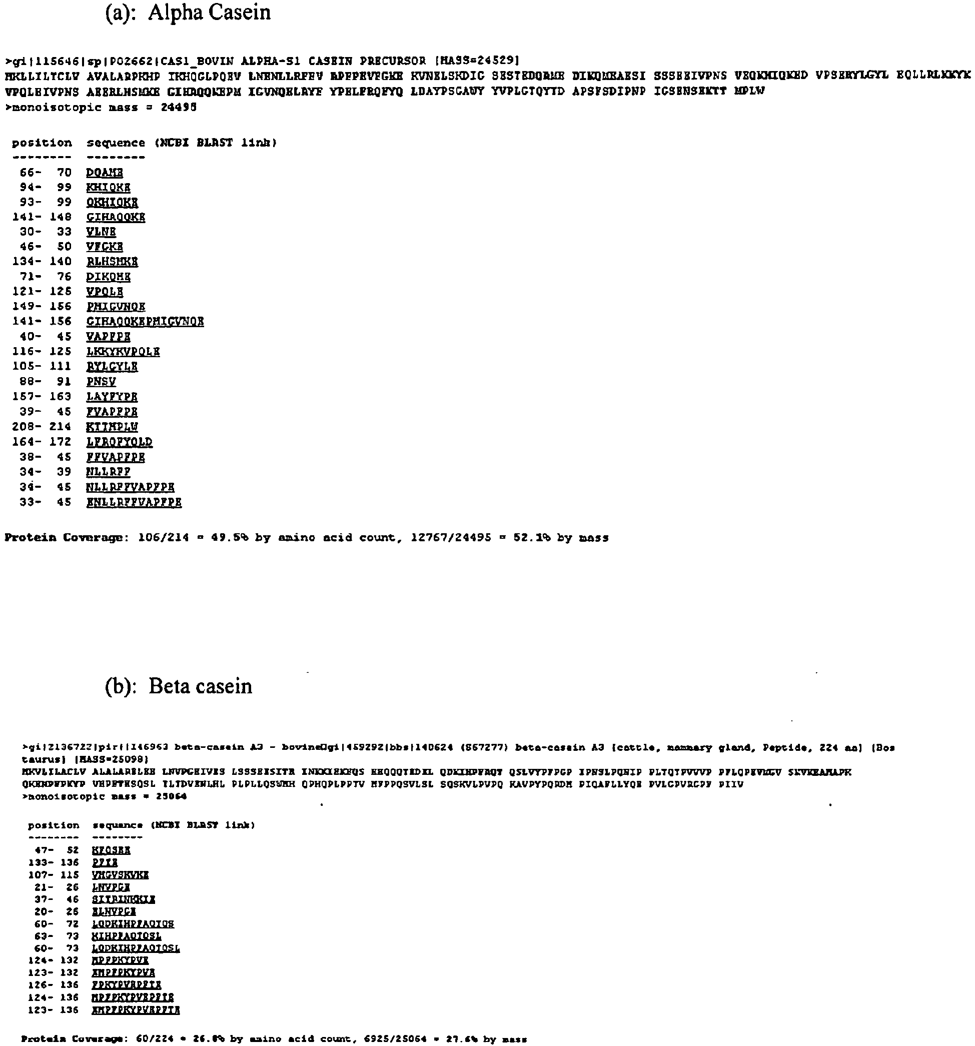

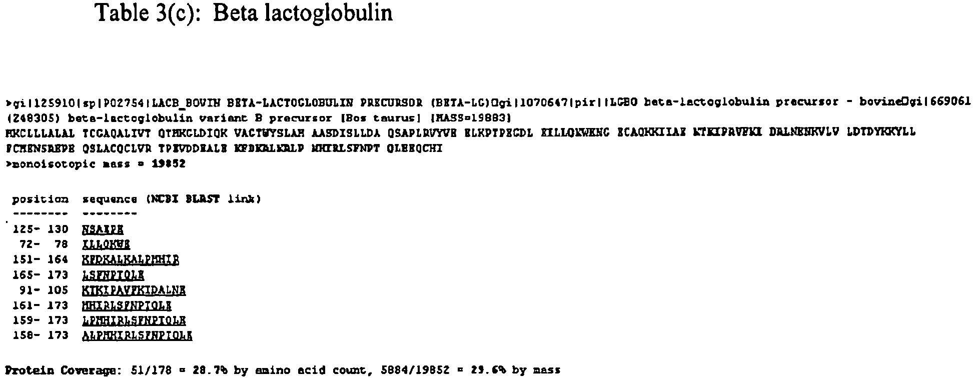

Similarly, the data obtained from the Mascot analysis of the peptide data for skim milk sample II digested for 20 hr are shown below in Tables 4 (a) for S-Glu, and 4 (b), and (c) for L-Glu, respectively.

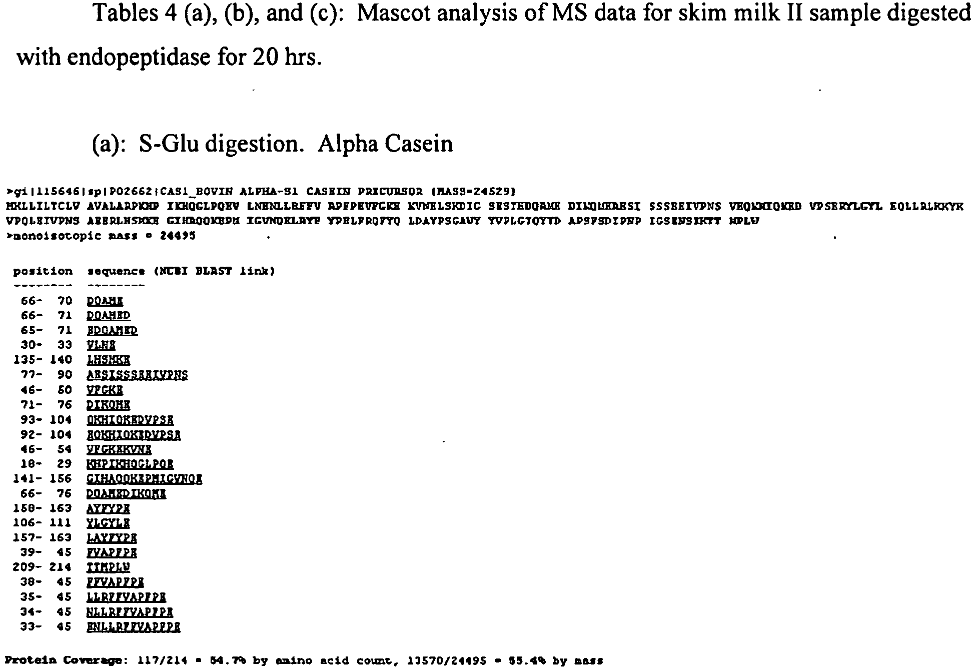

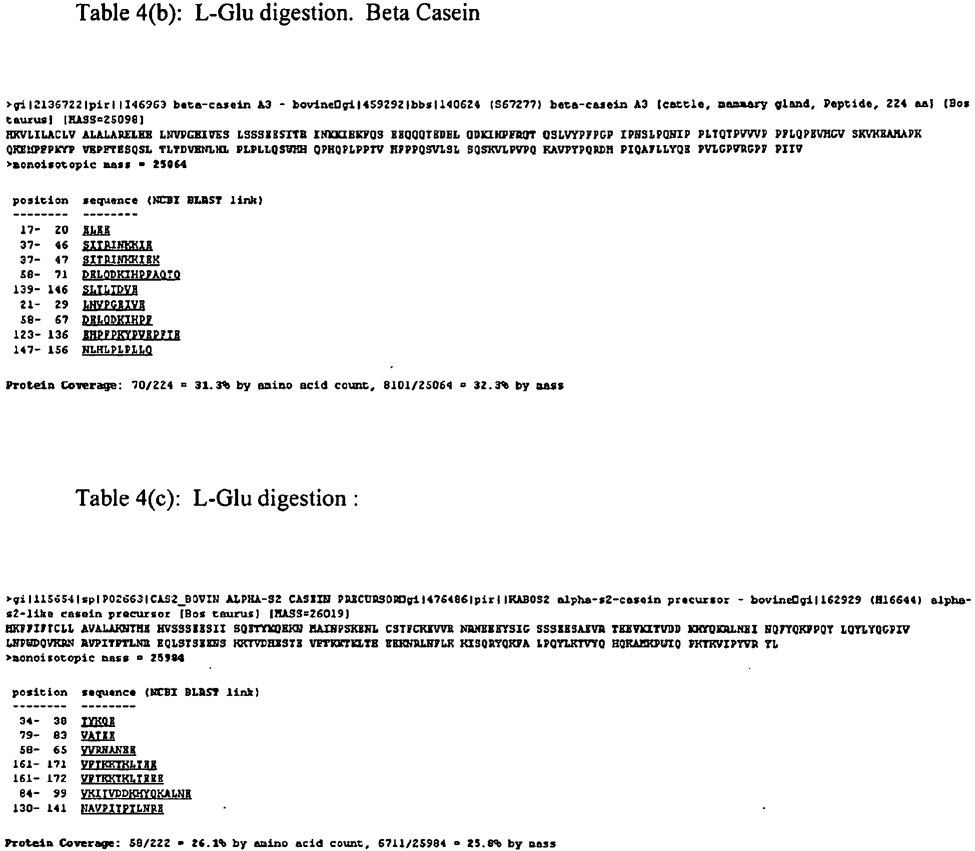

### Example 3

### Digestion of casein with endopeptidase activity

**Methods:** Ten (10) g of casein powder (Sigma, Catalog # C-5890) was mixed in 50 ml of 1 M phosphate buffer pH 6.5 and 200 ml of warm water. The contents were heated until the casein dissolved in the buffer. The casein solution was held at that temperature for five minutes. The casein solution was then cooled to room temperature, and adjusted to a final volume of 500 ml. Ten (10) ml of this casein solution was placed in each of eight 15 ml tubes and warmed up to 40 °C for 15 minutes in an incubator shaker. Each of three tubes received either endopeptidase S-Glu or L-Glu (500 µl of a 3.8 mg/ml stock solution). One tube each served as a control without enzyme added. Sample tubes were removed at t = 2, 4, and 20 hr, cooled, and centrifuged to separate precipitate and supernatant. Supernatants were tested for TP (total protein, g/l), % dry solid, and viscosity measurements. The supernatants were also analyzed electrophoretically using SDS-PAGE, to monitor the hydrolysis. Mass spectrometric (MS) analysis was performed to confirm the presence of individual proteolytic cleavage products. The results from the MS analysis were then used with the Mascot program, which allows peptide digest fragments to be used as the only data from which the source protein and its primary sequence can be identified using the public databases such as that maintained by the National Center for Biotechnology Information. More information on Mascot and its predecessor, Mowse, can be found on the world wide web, for example at the web site (www.matrixscience.com) maintained by Matrix Science Inc., (Boston, MA).

### Results:

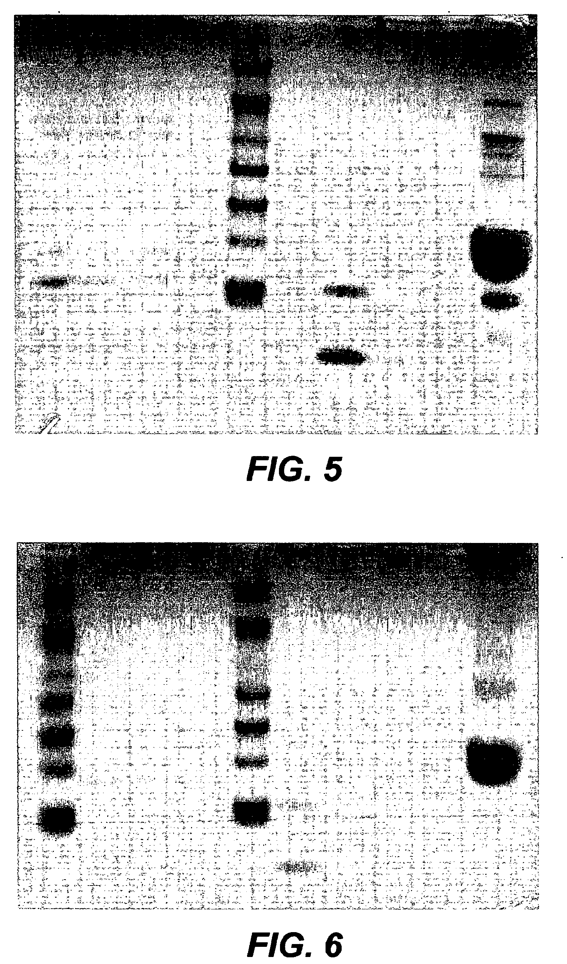

Buffered casein solution (16.85 g/l) treated with endopeptidase at the concentration of 0.19 mg of enzyme per g of casein solution, precipitated 58% casein within 2 hr using S-Glu. Some of the precipitated casein redissolved, however, due to further hydrolysis over time. The precipitated protein concentrations were thus 53% and 49% at 4 and 20 hr respectively. Treatment with L-Glu provided an increase in the precipitated casein over the time tested. At 2 hrs, 32.5% total protein (TP) was removed from casein solution. This increased to 34% by 4 hr; by 20 hrs, total protein removed was 54.2%. Figure 6 shows the results of the SDS-PAGE analysis of the casein digests.

The results of the Mascot analysis of the casein digests are shown below, in Table 5 and 6, for digestion results after 20 hrs with L-Glu and S-Glu enzymes, respectively.

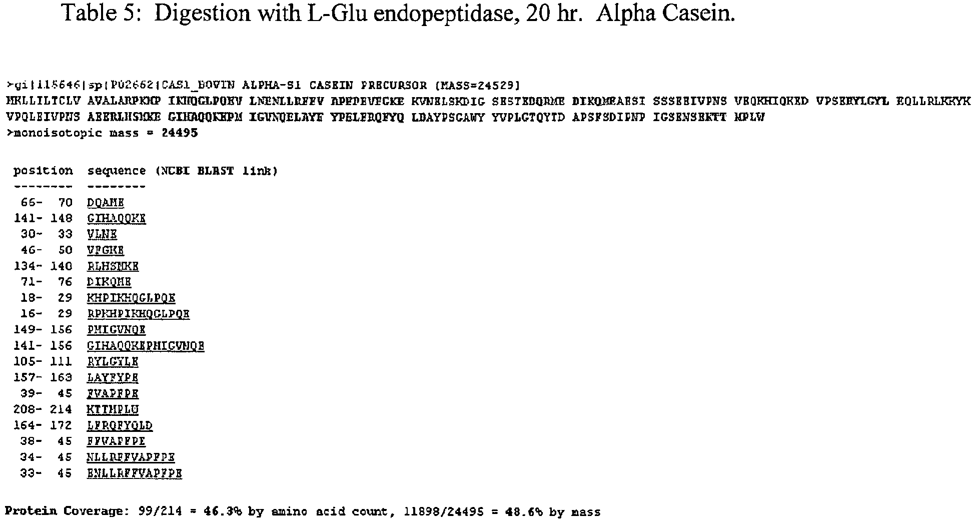

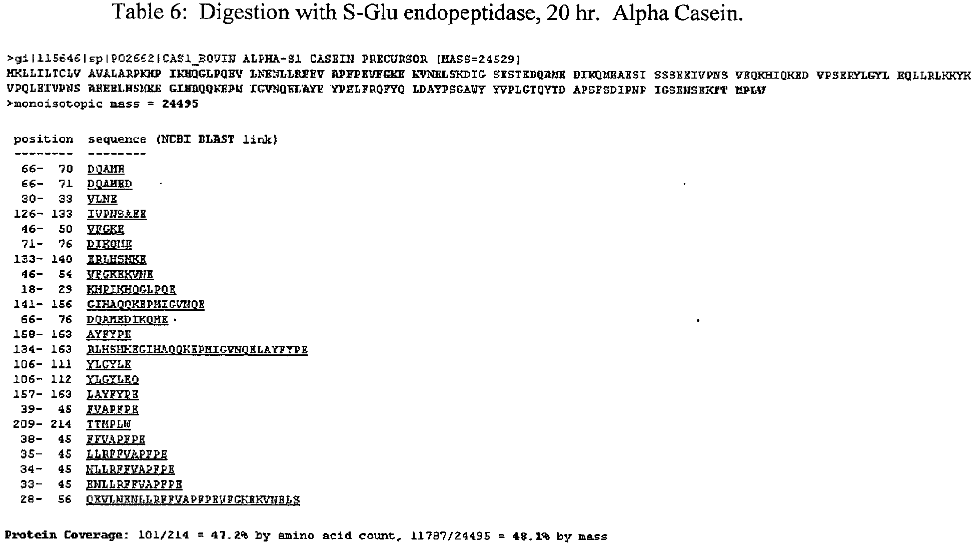

## Claims

1. A hydrolyzate comprising at least one milk protein hydrolyzed with at least one proteolytic enzyme that cleaves preferentially at a glutamic acid or aspartic acid residue and that cleaves one or more immunological determinants present in the protein, the hydrolyzate comprising at least a beta lactoglobulin hydrolyzed in the region between amino acids 55-98, the hydrolyzate having reduced immunogenicity in a subject predisposed to having an immune reaction to the protein and wherein the hydrolyzate is enriched in at least the peptide AQSAPLRVYVE (SEQ ID No. 1), wherein at least one proteolytic enzyme is an endopeptidase and wherein the endopeptidase is not one having the amino acid sequence

5 10 15 20 25 30
1 S V I G S D D R T R V T N T T A Y P Y R A I V H I S S S I G
31 S C T G W M I G P K T V A T A G H C I Y D T S S G S F A G T
61 A T V S P G R N G T S Y P Y G S V K S T R Y F I P S G W R S
91 G N T N Y D Y G A I E L S E P I G N T V G Y F G Y S Y T T S
121 S L V G T T V T I S G Y P G D K T A G T Q W Q H S G P I A I
151 S E T Y K L Q Y A M D T Y G G Q S G S P V F E Q S S S R T N
181 C S G P C S L A V H T N G V Y G G S S Y N R G T R I T K E V
211 F D N L T N W K N S A Q

2. The hydrolyzate of claim 1, wherein the endopeptidase preferentially cleaves on the carboxy-terminal side of a glutamic acid residue or an aspartic acid residue.

3. The hydrolyzate of claim 2, wherein the endopeptidase is UniProtKB/Swiss-Prot Primary Accession Number P39790.

4. A hydrolyzate comprising at least one milk protein hydrolyzed with at least one proteolytic enzyme that cleaves preferentially at a glutamic acid or aspartic acid residue, wherein the proteolytic enzyme is the endopeptidase UniProtKB/Swiss-Prot Primary Accession Number P39790, the hydrolyzate having reduced immunogenicity in a subject predisposed to having an immune reaction to the protein and wherein the hydrolyzate is enriched in at least the peptide AQSAPLRVYVE (SEQ ID No. 1).

5. The hydrolyzate of any one of claims 3-4 wherein the endopeptidase is inhibited by phenylmethylsulfonylfluoride or diisopropylfluorophosphate.

6. The hydrolyzate of claim 1, wherein the protein is a soluble, non-micellar protein.

7. The hydrolyzate of claim 6, wherein the protein is whey protein.

8. The hydrolyzate of claim 7, wherein the endopeptidase does not require a metal ion for its activity.

9. The hydrolyzate of claim 7, wherein the protein includes at least one of a lactalbumin, preferably an alpha lactalbumin.

10. The hydrolyzate of claim 9, wherein the protein is at least one of a lactalbumin or a lactoglobulin, preferably an alpha lactalbumin or a beta lactoglobulin.

11. The hydrolyzate of claim 10, wherein the hydrolyzate is enriched in one or more of the peptides ILLQKWE (SEQ ID NO: 2), KTKIPAVFKID (SEQ ID NO: 3), or KTKIPAVFKIDALNE (SEQ ID NO: 4).

12. The hydrolyzate of claim 1, that is enriched in peptides terminating in a glutamic acid or aspartic acid residue, as compared to the unhydrolyzated-protein.

13. The hydrolyzate of claim 12, that is enriched in one or more of the peptides ELEE (SEQ ID NO: 5), VATEE (SEQ ID NO: 6), PFTE (SEQ ID NO: 7), NSAEPE (SEQ ID NO: 8), VFGKE (SEQ ID NO: 9), ILLQKWE (SEQ ID NO: 2), KTKIPAVFKID (SEQ ID NO: 3), KTKIPAVFKIDALNE (SEQ ID NO: 4), IQPTPE (SEQ ID NO: 10), LKPTPE (SEQ ID NO: 11), LKPTPEGD (SEQ ID NO: 12), LKPTPEGDLE (SEQ ID NO: 13), TKIPAVFKID (SEQ ID NO: 14), or TKIPAVFKIDALNE (SEQ ID NO: 15).

14. The hydrolyzate of claim 12, that is enriched in one or more of the peptides DQAME (SEQ ID NO: 16), GIHAQQKE (SEQ ID NO: 17), VLNE (SEQ ID NO: 18), VFGKE (SEQ ID NO: 19), RLHSMKE (SEQ ID NO: 20), DIKQME (SEQ ID NO: 21), KHPIKHQGLPQE (SEQ ID NO: 22), RPKHPIKHQGLPQE (SEQ ID NO: 23), PMIGVNQE (SEQ ID NO: 24), GIHAQQKEPMEGVNQE (SEQ ID NO: 25), RYLGYLE (SEQ ID NO: 26), LAYFYPE (SEQ ID NO: 27), FVAPFPE (SEQ ID NO: 28), KTTMPLW (SEQ ID NO: 29), LFRQFYQLD (SEQ ID NO: 30), FFVAPFPE (SEQ ID NO: 31), NLLRFFV APFPE (SEQ ID NO: 32), or ENLLRFFVAPFPE (SEQ ID NO: 33).

15. A whey protein-containing hydrolyzate that is enriched in AQSAPLRVYVE (SEQ ID NO: 1).

16. The whey protein-containing hydrolyzate of claim 15 wherein the hydrolyzate is enriched in one or more of the peptides ILLQKWE (SEQ ID NO: 2), KTKIPAVFKID (SEQ ID NO: 3), or KTKIPAVFKIDALNE (SEQ ID NO: 4).

17. A food ingredient comprising a whey protein hydrolyzate enriched in AQSAPLRVYVE (SEQ ID NO: 1) having reduced immunogenicity in a subject predisposed to having an immune reaction to one or more whey proteins.

18. A food ingredient according to claim 17, wherein the hydrolyzate is enriched in one or more of the peptides ILLQKWE (SEQ ID NO: 2), KTKIP A VFKID (SEQ ID NO: 3), or KTKIPAVFKIDALNE (SEQ ID NO: 4).

19. A manufactured food product comprising a whey protein hydrolyzate enriched in AQSAPLRVYVE (SEQ ID NO: 1) having reduced immunogenicity in a subject predisposed to having an immune reaction to one or more whey proteins.

20. A manufactured food product according to claim 19, wherein the hydrolyzate is enriched in one or more of the peptides ILLQKWE (SEQ ID NO: 2), KTKIPAVFKID (SEQ ID NO: 3), or KTKIPAVFKIDALNE (SEQ ID NO: 4).

21. The manufactured food product of claim 19 or claim 20 that is an infant formula, a sports drink, a cheese-containing product, a protein supplement, a nutritional supplement, or a meal replacement product.

22. A non-food product that comprises the hydrolyzate of claim 1 or claim 4, wherein the nonfood product is a cosmetic, a lotion, or a cleanser for use on human skin.

23. A non-food product that comprises a hydrolyzate comprising at least one milk protein hydrolyzed with at least one proteolytic enzyme that cleaves preferentially at a glutamic acid or aspartic acid residue the hydrolyzate comprising at least a beta-lactoglobulin hydrolyzated in the region between amino acids 55-98 the hydrolyzate having reduced immunogenicity in a subject predisposed to having an immune reaction to the protein and wherein the hydrolyzate is enriched in at least the peptideAQSAPLRVYVE (SEQ ID No. 1).

24. The non-food product of claim 22 or claim 23 comprising a whey protein hydrolyzate enriched in one or more of the peptides AQSAPLRVYVE (SEQ ID NO: 1), ILLQKWE (SEQ ID NO: 2), KTKIPAVFKID (SEQ ID NO: 3), or KTKIPAVFKIDALNE (SEQ ID NO: 4), having reduced immunogenicity in a subject predisposed to having an immune reaction to one or more whey proteins.

25. A composition comprising at least two hydrolyzates of claim 1 or claim 4, wherein each hydrolyzate is produced with at least one different enzyme having a different specificity.

26. A hydrolyzate according to claim 2 wherein the at least one proteolytic enzyme comprises a further endopeptidase.

27. A hydrolyzate according to claim 26 wherein the further endopeptidase is UniProt KB/Swiss-Prot Primary Accession Number P80057.

28. Use of at least one proteolytic enzyme that cleaves preferentially at a glutamic acid or aspartic acid residue and that cleaves one or more immunological determinants present in at least one milk protein and wherein said at least one proteolytic enzyme is an endopeptidase and wherein the endopeptidase is not one having the amino acid sequence 
5 10 15 20 25 30
1 S V I G S D D R T R V T N T T A Y P Y R A I V H I S S S I G
31 S C T G W M I G P K T V A T A G H C I Y D T S S G S F A G T
61 A T V S P G R N G T S Y P Y G S V K S T R Y F I P S G W R S
91 G N T N Y D Y G A I E L S E P I G N T V G Y F G Y S Y T T S
121 S L V G T T V T I S G Y P G D K T A G T Q W Q H S G P I A I
151 S E T Y K L Q Y A M D T Y G G Q S G S P V F E Q S S S R T N
181 C S G P C S L A V H T N G V Y G G S S Y N R G T R I T K E V
211 F D N L T N W K N S A Q
 in the manufacture of a hydrolyzate comprising at least one milk protein hydrolyzed with at least one proteolytic enzyme which hydrolyzate comprises at least a beta lactoglobulin hydrolyzed in the region between amino acids 55-98 and wherein the hydrolyzate is enriched in at least the peptide AQSAPLRVYVE (SEQ ID No. 1) for reducing immunogenicity in a subject predisposed to having an immune reaction to the protein.

## Patentansprüche

1. Hydrolysat, umfassend wenigstens ein mit wenigstens einem proteolytischen Enzym, das vorzugsweise an einem Glutaminsäure- oder Asparaginsäurerest spaltet und das eine oder mehrere in dem Protein vorliegende immunologische Determinanten spaltet, hydrolysiertes Milchprotein, wobei das Hydrolysat wenigstens ein im Bereich zwischen Aminosäuren 55-98 hydrolysiertes Beta-Lactoglobulin umfasst, wobei das Hydrolysat eine reduzierte Immunogenität bei einem Individuum mit einer Veranlagung zu einer Immunreaktion gegen das Protein aufweist und wobei das Hydrolysat wenigstens an dem Peptid AQSAPLRVYVE (SEQ ID NO: 1) angereichert ist, wobei es sich bei wenigstens einem proteolytischen Enzym um eine Endopeptidase handelt und wobei die Endopeptidase nicht die Aminosäuresequenz 
5         10         15         20         25         30
1 S V I G S D D R T R V T N T T A Y P Y R A I V H I S S S I G
31 S C T G W M I G P K T V A T A G H C I Y D T S S G S F A G T
61 A T V S P G R N G T S Y P Y G S V K S T R Y F I P S G W R S
91 G N T N Y D Y G A I E L S E P I G N T V G Y F G Y S Y T T S
121 S L V G T T V T I S G Y P G D K T A G T Q W Q H S G P I A I
151 S E T Y K L Q Y A H D T Y G G Q S G S P V F E Q S S S R T N
181 C S G P C S L A V H T N G V Y G G S S Y N R G T R I T K E V
211 F D N L T N W K N S A Q
 aufweist.

2. Hydrolysat nach Anspruch 1, wobei die Endopeptidase vorzugsweise auf der carboxyterminalen Seite eines Glutaminsäurerests oder eines Asparaginsäurerests spaltet.

3. Hydrolysat nach Anspruch 2, wobei es sich bei der Endopeptidase um UniProtKB/Swiss-Prot Primary Accession Number P39790 handelt.

4. Hydrolysat, umfassend wenigstens ein mit wenigstens einem proteolytischen Enzym, das vorzugsweise an einem Glutaminsäure- oder Asparaginsäurerest spaltet, hydrolysiertes Milchprotein, wobei es sich bei dem proteolytischen Enzym um die Endopeptidase UniProtKB/Swiss-Prot Primary Accession Number P39790 handelt, wobei das Hydrolysat eine reduzierte Immunogenität bei einem Individuum mit einer Veranlagung zu einer Immunreaktion gegen das Protein aufweist und wobei das Hydrolysat wenigstens an dem Peptid AQSAPLRVYVE (SEQ ID NO: 1) angereichert ist.

5. Hydrolysat nach einem der Ansprüche 3-4, wobei die Endopeptidase durch Phenylmethylsulfonylfluorid oder Diisopropylfluorphosphat gehemmt wird.

6. Hydrolysat nach Anspruch 1, wobei es sich bei dem Protein um ein lösliches, nicht micellares Protein handelt.

7. Hydrolysat nach Anspruch 6, wobei es sich bei dem Protein um Molkenprotein handelt.

8. Hydrolysat nach Anspruch 7, wobei die Endopeptidase kein Metallion für ihre Aktivität benötigt.

9. Hydrolysat nach Anspruch 7, wobei das Protein wenigstens eines aus einem Lactalbumin enthält, vorzugsweise ein Alpha-Lactalbumin.

10. Hydrolysat nach Anspruch 9, wobei es sich bei dem Protein um wenigstens eines aus einem Lactalbumin oder einem Lactoglobulin handelt, vorzugsweise ein Alpha-Lactalbumin oder ein Beta-Lactoglobulin.

11. Hydrolysat nach Anspruch 10, wobei das Hydrolysat an einem oder mehreren der Peptide ILLQKWE (SEQ ID NO: 2), KTKIPAVFKID (SEQ ID NO: 3) oder KTKIPAVFKIDALNE (SEQ ID NO: 4) angereichert ist.

12. Hydrolysat nach Anspruch 1, das verglichen mit dem unhydrolysierten Protein an in einem Glutaminsäure- oder Asparaginsäurerest endenden Peptiden angereichert ist.

13. Hydrolysat nach Anspruch 12, das an einem oder mehreren der Peptide ELEE (SEQ ID NO: 5), VATEE (SEQ ID NO: 6), PFTE (SEQ ID NO: 7), NSAEPE (SEQ ID NO: 8), VFGKE (SEQ ID NO: 9), ILLQKWE (SEQ ID NO: 2), KTKIPAVFKID (SEQ ID NO: 3), KTKIPAVFKIDALNE (SEQ ID NO: 4), IQPTPE (SEQ ID NO: 10), LKPTPE (SEQ ID NO: 11), LKPTPEGD (SEQ ID NO: 12), LKPTPEGDLE (SEQ ID NO: 13), TKIPAVFKID (SEQ ID NO: 14) oder TKIPAVFKIDALNE (SEQ ID NO: 15) angereichert ist.

14. Hydrolysat nach Anspruch 12, das an einem oder mehreren der Peptide DQAME (SEQ ID NO: 16), GIHAQQKE (SEQ ID NO: 17), VLNE (SEQ ID NO: 18), VFGKE (SEQ ID NO: 19), RLHSMKE (SEQ ID NO: 20), DIKQME (SEQ ID NO: 21), KHPIKHQGLPQE (SEQ ID NO: 22), RPKHPIKHQGLPQE (SEQ ID NO: 23), PMIGVNQE (SEQ ID NO: 24), GIHAQQKEPMEGVNQE (SEQ ID NO: 25), RYLGYLE (SEQ ID NO: 26), LAYFYPE (SEQ ID NO: 27), FVAPFPE (SEQ ID NO: 28), KTTMPLW (SEQ ID NO: 29), LFRQFYQLD (SEQ ID NO: 30), FFVAPFPE (SEQ ID NO: 31), NLLRFFVAPFPE (SEQ ID NO: 32) oder ENLLRFFVAPFPE (SEQ ID NO: 33) angereichert ist.

15. Molkenproteinhaltiges Hydrolysat, das an AQSAPLRVYVE (SEQ ID NO: 1) angereichert ist.

16. Molkenproteinhaltiges Hydrolysat nach Anspruch 15, wobei das Hydrolysat an einem oder mehreren der Peptide ILLQKWE (SEQ ID NO: 2), KTKIPAVFKID (SEQ ID NO: 3) oder KTKIPAVFKIDALNE (SEQ ID NO: 4) angereichert ist.

17. Nahrungsmittelzutat, umfassend ein an AQSAPLRVYVE (SEQ ID NO: 1) angereichertes Molkenproteinhydrolysat mit reduzierter Immunogenität bei einem Individuum mit einer Veranlagung zu einer Immunreaktion gegen ein oder mehrere Molkenproteine.

18. Nahrungsmittelzutat gemäß Anspruch 17, wobei das Hydrolysat an einem oder mehreren der Peptide ILLQKWE (SEQ ID NO: 2), KTKIPAVFKID (SEQ ID NO: 3) oder KTKIPAVFKIDALNE (SEQ ID NO: 4) angereichert ist.

19. Nahrungsmittelerzeugnis, umfassend ein an AQSAPLRVYVE (SEQ ID NO: 1) angereichertes Molkenproteinhydrolysat mit reduzierter Immunogenität bei einem Individuum mit einer Veranlagung zu einer Immunreaktion gegen ein oder mehrere Molkenproteine.

20. Nahrungsmittelerzeugnis gemäß Anspruch 19, wobei das Hydrolysat an einem oder mehreren der Peptide ILLQKWE (SEQ ID NO: 2), KTKIPAVFKID (SEQ ID NO: 3) oder KTKIPAVFKIDALNE (SEQ ID NO: 4) angereichert ist.

21. Nahrungsmittelerzeugnis nach Anspruch 19 oder Anspruch 20, bei dem es sich um eine Säuglingsnahrung, ein Sportgetränk, ein käsehaltiges Produkt, ein Proteinergänzungsmittel, ein Nahrungsergänzungsmittel oder ein Fleischersatzprodukt handelt.

22. Nicht-Nahrungsmittelprodukt, das das Hydrolysat nach Anspruch 1 oder Anspruch 4 umfasst, wobei es sich bei dem Nicht-Nahrungsmittelprodukt um ein Kosmetikum, eine Lotion oder ein Reinigungsmittel zur Verwendung auf der menschlichen Haut handelt.

23. Nicht-Nahrungsmittelprodukt, das ein Hydrolysat, umfassend wenigstens ein mit wenigstens einem proteolytischen Enzym, das vorzugsweise an einem Glutaminsäure- oder Asparaginsäurerest spaltet, hydrolysiertes Milchprotein, umfasst, wobei das Hydrolysat wenigstens ein im Bereich zwischen Aminosäuren 55-98 hydrolysiertes Beta-Lactoglobulin umfasst, wobei das Hydrolysat eine reduzierte Immunogenität bei einem Individuum mit einer Veranlagung zu einer Immunreaktion gegen das Protein aufweist und wobei das Hydrolysat wenigstens an dem Peptid AQSAPLRVYVE (SEQ ID NO: 1) angereichert ist.

24. Nicht-Nahrungsmittelprodukt nach Anspruch 22 oder Anspruch 23, umfassend ein an einem oder mehreren der Peptide AQSAPLRVYVE (SEQ ID NO: 1), ILLQKWE (SEQ ID NO: 2), KTKIPAVFKID (SEQ ID NO: 3) oder KTKIPAVFKIDALNE (SEQ ID NO: 4) angereichertes Molkenproteinhydrolysat mit reduzierter Immunogenität bei einem Individuum mit einer Veranlagung zu einer Immunreaktion gegen ein oder mehrere Molkenproteine.

25. Zusammensetzung, umfassend wenigstens zwei Hydrolysate nach Anspruch 1 oder Anspruch 4, wobei die Hydrolysate jeweils mit wenigstens einem unterschiedlichen Enzym mit einer unterschiedlichen Spezifität hergestellt werden.

26. Hydrolysat gemäß Anspruch 2, wobei das wenigstens eine proteolytische Enzym eine weitere Endopeptidase umfasst.

27. Hydrolysat gemäß Anspruch 26, wobei es sich bei der weiteren Endopeptidase um UniProtKB/Swiss-Prot Primary Accession Number P80057 handelt.

28. Verwendung wenigstens eines proteolytischen Enzyms, das vorzugsweise an einem Glutaminsäure- oder Asparaginsäurerest spaltet und das eine oder mehrere in wenigstens einem Milchprotein vorliegende immunologische Determinanten spaltet, und wobei es sich bei dem wenigstens einen proteolytischen Enzym um eine Endopeptidase handelt und wobei die Endopeptidase nicht die Aminosäuresequenz 
S V I G S D D R T R V T N T T A Y P Y R A I V H I S S S I G
31 S C T G W M I G P K T V A T A G H C I Y D T S S G S F A G T
61 A T V S P G R N G T S Y P Y G S V K S T R Y F I P S G W R S
91 G N T N Y D Y G A I E L S E P I G N T V G Y F G Y S Y T T S
121 S L V G T T V T I S G Y P G D K T A G T Q W Q H S G P I A I
151 S E T Y K L Q Y A M D T Y G G Q S G S P V F E Q S S S R T N
181 C S G P C S L A V H T N G V Y G G S S Y N R G T R I T K E V
211 F D N L T N W K N S A Q
 aufweist, bei der Erzeugung eines Hydrolysats, umfassend wenigstens ein mit wenigstens einem proteolytischen Enzym hydrolysiertes Milchprotein, wobei das Hydrolysat wenigstens ein im Bereich zwischen Aminosäuren 55-98 hydrolysiertes Beta-Lactoglobulin umfasst und wobei das Hydrolysat wenigstens an dem Peptid AQSAPLRVYVE (SEQ ID NO: 1) angereichert ist, zur Reduzierung der Immunogenität bei einem Individuum mit einer Veranlagung zu einer Immunreaktion gegen das Protein.

## Revendications

1. Hydrolysat comprenant au moins une protéine de lait hydrolysée par au moins une enzyme protéolytique qui clive préférentiellement au niveau d'un résidu d'acide glutamique ou d'acide aspartique et qui clive un ou plusieurs déterminants immunologiques présents dans la protéine, l'hydrolysat comprenant au moins une bêta-lactoglobuline hydrolysée dans la région entre les acides aminés 55-98, l'hydrolysat ayant une immunogénicité réduite chez un sujet prédisposé à une réaction immunitaire à la protéine et **caractérisé en ce que** l'hydrolysat est enrichi en au moins le peptide AQSAPLRVYVE (SEQ ID n° 1), où au moins une enzyme protéolytique est une endopeptidase et où l'endopeptidase n'est pas une endopeptidase ayant la séquence d'acides aminés

5 10 15 20 25 30
1 S V I G S D D R T R V T N T T A Y P Y R A I V H I S S S I G
31 S C T G W M I G P K T V A T A G H C I Y D T S S G S F A G T
61 A T V S P G R N G T S Y P Y G S V K S T R Y F I P S G W R S
91 G N T N Y D Y G A I E L S E P I G N T V G Y F G Y S Y T T S
121 S L V G T T V T I S G Y P G D K T A G T Q W Q H S G P I A I
151 S E T Y K L Q Y A M D T Y G G Q S G S P V F E Q S S S R T N
181 C S G P C S L A V H T N G V Y G G S S Y N R G T R I T K E V
211 F D N L T N W K N S A Q

2. Hydrolysat selon la revendication 1, **caractérisé en ce que** l'endopeptidase clive préférentiellement au niveau de l'extrémité carboxy-terminale d'un résidu d'acide glutamique ou d'un résidu d'acide aspartique.

3. Hydrolysat selon la revendication 2, **caractérisé en ce que** l'endopeptidase est le Numéro d'Accession Primaire P39790 d'UniProtKB/Swiss-Prot.

4. Hydrolysat comprenant au moins une protéine de lait hydrolysée par au moins une enzyme protéolytique qui clive préférentiellement au niveau d'un résidu d'acide glutamique ou d'acide aspartique, **caractérisé en ce que** l'enzyme protéolytique est l'endopeptidase de Numéro d'Accession Primaire P39790 d'UniProtKB/Swiss-Prot, l'hydrolysat ayant une immunogénicité réduite chez un sujet prédisposé à une réaction immunitaire à la protéine et **caractérisé en ce que** l'hydrolysat est enrichi en au moins le peptide AQSAPLRVYVE (SEQ ID n° 1) .

5. Hydrolysat selon l'une quelconque des revendications 3-4, **caractérisé en ce que** l'endopeptidase est inhibée par le fluorure de phénylméthylsulfonyle ou le fluorophosphate de diisopropyle.

6. Hydrolysat selon la revendication 1, **caractérisé en ce que** la protéine est une protéine soluble non micellaire.

7. Hydrolysat selon la revendication 6, **caractérisé en ce que** la protéine est une protéine de lactosérum.

8. Hydrolysat selon la revendication 7, **caractérisé en ce que** l'endopeptidase ne nécessite pas d'ion métallique pour son activité.

9. Hydrolysat selon la revendication 7, **caractérisé en ce que** la protéine comporte au moins l'une parmi une lactalbumine, préférablement une alpha-lactalbumine.

10. Hydrolysat selon la revendication 9, **caractérisé en ce que** la protéine est au moins l'une parmi une lactalbumine ou une lactoglobuline, préférablement une alpha-lactalbumine ou une bêta-lactoglobuline.

11. Hydrolysat selon la revendication 10, **caractérisé en ce que** l'hydrolysat est enrichi en l'un ou plusieurs parmi les peptides ILLQKWE (SEQ ID n° 2), KTKIPAVFKID (SEQ ID n° 3), ou KTKIPAVFKIDALNE (SEQ ID n° 4).

12. Hydrolysat selon la revendication 1, qui est enrichi en peptides se terminant par un résidu d'acide glutamique ou d'acide aspartique, par rapport à la protéine non hydrolysée.

13. Hydrolysat selon la revendication 12, qui est enrichi en l'un ou plusieurs parmi les peptides ELEE (SEQ ID n° 5), VATEE (SEQ ID n° 6), PFTE (SEQ ID n° 7), NSAEPE (SEQ ID n° 8), VFGKE (SEQ ID n° 9), ILLQKWE (SEQ ID n° 2), KTKIPAVFKID (SEQ ID n° 3), KTKIPAVFKIDALNE (SEQ ID n° 4), IQPTPE (SEQ ID n° 10), LKPTPE (SEQ ID n° 11), LKPTPEGD (SEQ ID n° 12), LKPTPEGDLE (SEQ ID n° 13), TKIPAVFKID (SEQ ID n° 14), ou TKIPAVFKIDALNE (SEQ ID n° 15).

14. Hydrolysat selon la revendication 12, qui est enrichi en l'un ou plusieurs parmi les peptides DQAME (SEQ ID n° 16), GIHAQQKE (SEQ ID n° 17), VLNE (SEQ ID n° 18), VFGKE (SEQ ID n° 19), RLHSMKE (SEQ ID n° 20), DIKQME (SEQ ID n° 21), KHPIKHQGLPQE (SEQ ID n° 22), RPKHPIKHQGLPQE (SEQ ID n° 23), PMIGVNQE (SEQ ID n° 24), GIHAQQKEPMEGVNQE (SEQ ID n° 25), RYLGYLE (SEQ ID n° 26), LAYFYPE (SEQ ID n° 27), FVAPFPE (SEQ ID n° 28), KTTMPLW (SEQ ID n° 29), LFRQFYQLD (SEQ ID n° 30), FFVAPFPE (SEQ ID n° 31), NLLRFFVAPFPE (SEQ ID n° 32), ou ENLLRFFVAPFPE (SEQ ID n° 33).

15. Hydrolysat contenant des protéines de lactosérum qui est enrichi en AQSAPLRVYVE (SEQ ID n° 1).

16. Hydrolysat contenant des protéines de lactosérum selon la revendication 15, **caractérisé en ce que** l'hydrolysat est enrichi en l'un ou plusieurs parmi les peptides ILLQKWE (SEQ ID n° 2), KTKIPAVFKID (SEQ ID n° 3), ou KTKIPAVFKIDALNE (SEQ ID n° 4).

17. Ingrédient alimentaire comprenant un hydrolysat de protéines de lactosérum enrichi en AQSAPLRVYVE (SEQ ID n° 1) ayant une immunogénicité réduite chez un sujet prédisposé à une réaction immunitaire à une ou plusieurs protéines de lactosérum.

18. Ingrédient alimentaire selon la revendication 17, **caractérisé en ce que** l'hydrolysat est enrichi en l'un ou plusieurs parmi les peptides ILLQKWE (SEQ ID n° 2), KTKIPAVFKID (SEQ ID n° 3), ou KTKIPAVFKIDALNE (SEQ ID n° 4).

19. Produit alimentaire manufacturé comprenant un hydrolysat de protéines de lactosérum enrichi en AQSAPLRVYVE (SEQ ID n° 1) ayant une immunogénicité réduite chez un sujet prédisposé à une réaction immunitaire à une ou plusieurs protéines de lactosérum.

20. Produit alimentaire manufacturé selon la revendication 19, **caractérisé en ce que** l'hydrolysat est enrichi en l'un ou plusieurs parmi les peptides ILLQKWE (SEQ ID n° 2), KTKIPAVFKID (SEQ ID n° 3), ou KTKIPAVFKIDALNE (SEQ ID n° 4).

21. Produit alimentaire manufacturé selon la revendication 19 ou la revendication 20, **caractérisé en ce qu'**il s'agit d'une préparation lactée pour nourrisson, d'une boisson pour sportifs, d'un produit à base de fromage, d'un complément protéique, d'un complément nutritionnel, ou d'un produit de substitut de repas.

22. Produit non alimentaire comprenant l'hydrolysat selon la revendication 1 ou la revendication 4, **caractérisé en ce que** le produit non alimentaire est un produit cosmétique, une lotion ou un nettoyant pour une utilisation sur la peau humaine.

23. Produit non alimentaire comprenant un hydrolysat comprenant au moins une protéine de lait hydrolysée par au moins une enzyme protéolytique qui clive préférentiellement au niveau d'un résidu d'acide glutamique ou d'acide aspartique, l'hydrolysat comprenant au moins une bêta-lactoglobuline hydrolysée dans la région entre les acides aminés 55-98, l'hydrolysat ayant une immunogénicité réduite chez un sujet prédisposé à une réaction immunitaire à la protéine et **caractérisé en ce que** l'hydrolysat est enrichi en au moins le peptide AQSAPLRVYVE (SEQ ID n° 1) .

24. Produit non alimentaire selon la revendication 22 ou la revendication 23, comprenant un hydrolysat de protéines de lactosérum enrichi en l'un ou plusieurs parmi les peptides AQSAPLRVYVE (SEQ ID n° 1), ILLQKWE (SEQ ID n° 2), KTKIPAVFKID (SEQ ID n° 3), ou KTKIPAVFKIDALNE (SEQ ID n° 4), ayant une immunogénicité réduite chez un sujet prédisposé à une réaction immunitaire à une ou plusieurs protéines de lactosérum.

25. Composition comprenant au moins deux hydrolysats selon la revendication 1 ou la revendication 4, **caractérisée en ce que** chaque hydrolysat est produit avec au moins une enzyme différente ayant une spécificité différente.

26. Hydrolysat selon la revendication 2, **caractérisé en ce que** la au moins une enzyme protéolytique comprend une autre endopeptidase.

27. Hydrolysat selon la revendication 26, **caractérisé en ce que** l'autre endopeptidase est le Numéro d'Accession Primaire P80057 d'UniProtKB/Swiss-Prot.

28. Utilisation d'au moins une enzyme protéolytique qui clive préférentiellement au niveau d'un résidu d'acide glutamique ou d'acide aspartique et qui clive un ou plusieurs déterminants immunologiques présents dans au moins une protéine de lait, et où ledit au moins une enzyme protéolytique est une endopeptidase et où l'endopeptidase n'est pas une endopeptidase ayant la séquence d'acides aminés 
5    10    15    20    25    30
1 S V I G S D D R T R V T N T T A Y P Y R A I V H I S S S I G
31 S C T G W M I G P K T V A T A G H C I Y D T S S G S F A G T
61 A T V S P G R N G T S Y P Y G S V K S T R Y F I P S G W R S
91 G N T N Y D Y G A I E L S E P I G N T V G Y F G Y S Y T T S
121 S L V G T T V T I S G Y P G D K T A G T Q W Q H S G P I A I
151 S E T Y K L Q Y A M D T Y G G Q S G S P V F E Q S S S R T N
181 C S G P C S L A V H T N G V Y G G S S Y N R G T R I T K E V
211 F D N L T N W K N S A Q
 dans la fabrication d'un hydrolysat comprenant au moins une protéine de lait hydrolysée par au moins une enzyme protéolytique, lequel hydrolysat comprend au moins une bêta-lactoglobuline hydrolysée dans la région entre les acides aminés 55-98 et où l'hydrolysat est enrichi en au moins le peptide AQSAPLRVYVE (SEQ ID n° 1), afin de réduire l'immunogénicité chez un sujet prédisposé à une réaction immunitaire à la protéine.
